# EUROPÄISCHE PATENTSCHRIFT

(11) **EP 2 322 261 B1**
(45) Veröffentlichungstag und Bekanntmachung des Hinweises auf die Patenterteilung: **21.12.2016**
(21) Anmeldenummer: 10190346.6
(22) Anmeldetag: 08.11.2010
(51) Int. Cl.: B01D 3/14, B01D 3/32, C07C 68/06, C07C 68/08, C07C 69/96, C07B 61/00

(54) **Verfahren zur Reinigung von Dialkylcarbonaten**
Method for cleaning dialkyl carbonates
Procédé destiné au nettoyage de carbonates de dialkyle

(30) Priorität: 14.11.2009 DE 102009053370
(43) Veröffentlichungstag der Anmeldung: 18.05.2011
(73) Patentinhaber: Covestro Deutschland AG, 51373 Leverkusen (DE)
(72) Erfinder: Ooms, Pieter, 47800 Krefeld (DE); Risse, Friedhelm, 50739 Köln (DE); Düx, Andre, 50321 Brühl (DE); Buchaly, Carsten, 40233 Düsseldorf (DE); Pancur, Thomas, 24161 Altenholz (DE); Susanto, Arthur, 50670 Köln (DE); Ronge, Georg, 40549 Düsseldorf (DE); Vanden Eynde, Johan, 9052 Zwijnaarde (BE); Wuytack, Wim, 9160 Lokeren (BE)
(74) Vertreter: Levpat

(56) Entgegenhaltungen:
- EP-A1- 2 239 249
- US-A1- 2008 293 960
- US-A1- 2009 076 293

## Beschreibung

Gegenstand der vorliegenden Erfindung ist ein spezielles Verfahren zur Reinigung von Dialkylcarbonaten. Insbesondere betrifft die vorliegende Erfindung ein kontinuierliches Verfahren zur Aufreinigung eines Dialkylcarbonat / Alkylalkohol-Gemisches bei der Herstellung von Dialkylcarbonat durch katalysierte Umesterung eines zyklischen Alkylencarbonats (z.B. Ethylen- oder Propylencarbonat) mit Alkylalkoholen. Zur Optimierung der Wirtschaftlichkeit und Energieeffizienz des Verfahrens wird eine Vorrichtung zur Zwischenerhitzung des apparateinternen Flüssigkeitstromes eingesetzt.

Die Aufreinigung von Dialkylcarbonat ist als Vorläufer des Diarylcarbonats aufgrund der hohen Anforderungen an deren Reinheit für die Herstellung von qualitativ hochwertigen Polycarbonaten mittels Schmelzeumesterung von großer Bedeutung. Dialkylcarbonate hergestellt aus Umesterung von zyklischem Alkylencarbonat und Alkylalkohol können sowohl hochsiedende als auch leichtsiedende Komponenten sowie Katalysatorreste als Verunreinigungen enthalten. Hochsiedende Komponenten - oft auch als Schwersieder bezeichnet - im Sinne dieses Aufreinigungsverfahrens sind solche, deren Siedepunkt oberhalb desjenigen des Dialkylcarbonats liegt. Leichtsiedende Komponenten - oft auch als Leichtsieder bezeichnet - im Sinne dieses Aufreinigungsverfahrens sind solche, deren Siedepunkt unterhalb desjenigen des Dialkylcarbonats liegt. All diese Verunreinigungen führen zu erheblicher qualitativer Beeinträchtigung der herzustellenden Diarylcarbonate sowie anschließend daraus hergestellten Polycarbonate und müssen vor der Weiterverarbeitung der Dialkylcarbonate durch entsprechende Aufreinigung entfernt werden.

Die Herstellung von Dialkylcarbonaten aus zyklischem Alkylencarbonat und Alkylalkohol, bei der gleichzeitig Alkylenglykol als Nebenprodukt entsteht, ist bekannt und vielfach beschrieben worden. In US-6,930,195 B beispielsweise, wurde diese katalysierte Umesterungsreaktion als zweistufige Gleichgewichtsreaktion beschrieben. In der ersten Reaktionsstufe reagiert das zyklische Alkylencarbonat mit Alkohol zu Hydroxy-Alkylcarbonat als Zwischenprodukt. Das Zwischenprodukt wird dann in der zweiten Reaktionsstufe mit Hilfe von Alkylalkohol umgesetzt zu den Produkten: Dialkylcarbonat und Alkylenglykol.

Für die technische Realisierung des Dialkylcarbonat-Herstellungsverfahrens hat sich die Verwendung einer reaktiven Destillationskolonne (im Folgenden auch Umesterungskolonne genannt), die unter anderem bereits in EP 530 615 A, EP 569 812 A und EP 1 086 940 A beschrieben wurde, als besonders günstig erwiesen. In EP 569 812 A wird das zyklische Alkylencarbonat in den oberen Teil der Umesterungskolonne und der Dialkylcarbonat enthaltende Alkylalkohol in den mittleren oder unteren Teil der Umesterungskolonne kontinuierlich eingeführt. Zusätzlich wird unterhalb der Einführung des Dialkylcarbonat enthaltenden Alkylalkohols nahezu reiner Alkylalkohol eingeführt. Das Schwersiedergemisch, welches das hergestellte Alkylenglykol als Nebenprodukt beinhaltet, wird am Sumpf der Umesterungskolonne kontinuierlich abgezogen. Das Leichtsiedergemisch, welches das hergestellte Dialkylcarbonat beinhaltet, wird am Kopf der Umesterungskolonne als Dialkylcarbonat-Alkylalkohol-Gemisch abgezogen und einem weiteren Aufreinigungsschritt unterworfen.

Im Rahmen dieser Anmeldung versteht man unter "nahezu rein" auch ein Gemisch enthaltend ≥ 99 % an Hauptkomponente.

Die Destillationskolonne für die Aufreinigung des Dialkylcarbonat- Alkylalkohol-Gemisches wird bei einem höheren Druck betrieben, so dass ein weiteres Dialkylcarbonat- Alkylalkohol-Gemisch mit einem niedrigeren Dialkylcarbonat-Anteil am Kolonnenkopf abgezogen werden kann. Das Dialkylcarbonat als Hauptprodukt wird am Sumpf dieser Aufreinigungskolonne gewonnen.

Für die Entwicklung eines wirtschaftlich attraktiven Herstellungsverfahrens für Dialkylcarbonate spielen viele Faktoren eine wichtige Rolle. Die meisten Literaturquellen beschäftigen sich mit den Reaktionsparametern, wie z.B. Umsatz, Selektivität oder auch Produktreinheit. Seltener wurde die Energieeffizienz des Verfahrens thematisiert (z.B. EP 569 812 A, J P 2003-104937, WO 2007/096340, WO 2007/096343), obwohl diese Faktoren nicht unerheblich zu der wirtschaftlichen Attraktivität des Verfahrens beitragen. Daher werden in dieser Erfindung Maßnahmen eingeführt, um die Energieeffizienz des Verfahrens zu erhöhen.

In EP 569 812 A wird der Energieeinsatz in der Herstellung des Dialkylcarbonates dadurch reduziert, dass viele verfahrensinterne Ströme nicht kondensiert sondern als dampfförmige Ströme geführt werden.

In WO 2007/096340 wird ein Verfahren beschrieben, in dem Alkylencarbonat aus Alkylenoxid und CO₂ erzeugt und anschließend das Alkylencarbonat mit Alkylalkohol zu Dialkylcarbonat und Alkylenglykol umgesetzt wird, wobei das im zweiten Schritt entstehende Gemisch, welches Dialkylcarbonat und Alkylenglykol enthält, aufgereinigt wird. Die Reaktion zum Alkylencarbonat ist exotherm und der entsprechende Alkylencarbonat-Produktstrom wird zur Aufwärmung des Dialkylcarbonat-Alkylenglykol-Produktstromes in der Aufreinigung eingesetzt.

In WO 2007/096343 wird das in einer Umesterungskolonne aus Alkylencarbonat und Alkylalkohol entstehende Gemisch aus Dialkylcarbonat und Alkylalkohol mittels Extraktivdestillation gereinigt, wobei Alkylencarbonat als Extraktionsmittel dient. Nachdem das Dialkylcarbonat destillativ vom Extraktionsmittel getrennt wurde, wird mit dem heißen Sumpfablauf dieser Kolonne, der das Extraktionsmittel enthält, der der Umesterungskolonne zugeführte Alkylalkohol erwärmt.

In JP2003-104937 werden verschiedene Verfahrensvarianten zur Aufarbeitung eines Ethylencarbonat-Ethylenglykol-Gemisches und Bereitstellung des gereinigten Ethylencarbonates für das Verfahren zur Herstellung von Dimethylcarbonat auch unter dem Gesichtspunkt des Energieverbrauchs betrachtet.

Die EP 2 239 249 A1 und die US 2008/0293960 A1 beschreiben jeweils ein Verfahren zur Herstellung von Diarylcarbonaten und/oder Alkylarylcarbonaten aus Dialkylcarbonaten und aromatischen Hydroxyverbindungen unter Einsatz einer Umesterungskolonne. Am Kopf der Umesterungskolonne wird ein Gemisch aus Dialkylcarbonat und Alkylalkohol entnommen und einer Destillationskolonne zugeführt. Der Energiebedarf zum Betrieb des Sumpfverdampfers der Destillationskolonne ist verhältnismässig hoch.

Die US 2009/076293 A1 beschreibt ein Verfahren zur Herstellung von Diarylcarbonaten und/oder Alkylarylcarbonaten aus Dialkylcarbonaten und aromatischen Hydroxyverbindungen unter Einsatz von zwei Umesterungskolonnen. Am Kopf der Umesterungskolonnen wird ein Gemisch aus Dialkylcarbonat und Alkylalkohol entnommen und einer Destillationskolonne zugeführt. Gemäss diesem Dokument wird durch geeignete Wahl des Drucks in der Destillationskolonne eine direkte Energieintegration zwischen einem Kondensator der Umesterungskolonne und dem Sumpfverdampfer der Destillationskolonne möglich. Eine zusätzliche Energieintegration kann durch Nutzung der Kondensationsenergie eines Zwischenkondensators der Umesterungskolonne zur Erwärmung des Zustroms der Destillationskolonne erreicht werden.

Es stellt sich heraus, dass die Destillation des aus der Umesterungskolonne stammenden leichtsiedenden Produktstroms nach den Verfahren des Standes der Technik nur mit geringer Energieeffizienz und apparativ aufwändig durchgeführt werden kann. Eine Vorrichtung zur Reduzierung des Energieeintrags ist bisher nicht bekannt.

Es bestand demnach Bedarf, ein Verfahren zur Aufreinigung von Dialkylcarbonaten, bereitzustellen, das die vorangehend genannten Nachteile nicht aufweist und in welchem gegenüber den vorangehend genannten bekannten Verfahren eine Energieintegration in effizienter Weise möglich ist bzw. eine verbesserte Energieintegration erreicht werden kann. Weiterhin bestand Bedarf an einem Verfahren zur Aufreinigung von Dialkylcarbonaten bei dem mit möglichst einfacher apparativer und günstiger energetischer Ausführung gegebenenfalls auch mittelsiedende Nebenkomponenten als Verunreinigungen entfernt werden können, wobei unter mittelsiedenden Nebenkomponenten solche zu verstehen sind, deren Siedepunkt zwischen dem Siedepunkt des Alkylalkohols und dem des Dialkylcarbonats liegt.

Die Aufgabe, die der Erfindung zugrunde lag, bestand demnach darin, ein Verfahren zur Aufreinigung von Dialkylcarbonaten, bereitzustellen, welches gegenüber bekannten Verfahren einen verringerten Energieverbrauch aufweist.

Überraschend wurde nun gefunden, dass die destillative Aufreinigung von Dialkylcarbonaten auch bei geringem apparativen und energetischen Aufwand durchgeführt werden kann, wenn die bei der Destillation und / oder dem nachfolgenden Verfahren zur Herstellung von Diarylcarbonat anfallende Abwärme energetisch genutzt wird.

Insbesondere kann der Bedarf an Wärmeenergie auf dem Temperaturniveau Tₛᵥ, welches zum Betrieb des Sumpfverdampfers benötigt wird, besonders einfach und günstig durch die zusätzliche Verwendung einer technischen Vorrichtung zur Zwischenerhitzung in der Destillationskolonne verringert werden. Der Energieeintrag in diesem Zwischenerhitzer stammt bevorzugt von dem anschließenden Verfahren zur Herstellung von Diarylcarbonat. Bedingt durch die niedrigere Temperatur des internen Stoffstromes im Vergleich zur Sumpftemperatur der Kolonne, kann für die Zwischenerhitzung Wärmeenergie auf dem Temperaturniveau T_{z} mit T_{z} < Tₛᵥ eingesetzt werden. Dieses Konzept führt insgesamt zu einer Verringerung des Verbrauchs an Wärmeenergie auf einem Temperaturniveau größer oder gleich Tₛᵥ, weil nun die in anderen chemischen Herstellverfahren z. B. bei einer Kondensation oder bei der Abkühlung eines Stoffstromes anfallende Wärmeenergie auf einem Temperaturniveau kleiner Tₛᵥ sinnvoll genutzt werden kann und die Menge an im Allgemeinen kostenintensiverer Wärmeenergie auf einem Temperaturniveau größer oder gleich Tsv reduziert werden kann.

Die in anderen chemischen Herstellverfahren durch die Kondensation oder die Abkühlung eines Stoffstromes gewonnene Wärmeenergie auf dem Temperaturniveau Tz kann entweder direkt oder indirekt dem Zwischenerhitzer zugeführt. Bei direkter Zuführung erwärmt der Stoffstrom, der in den anderen chemischen Herstellverfahren kondensiert oder abgekühlt werden soll, mittels des Zwischenerhitzers den kolonneninternen Strom der Destillationskolonne zur Aufreinigung des Dialkylcarbonates. Bei indirekter Zuführung erwärmt der zu kondensierende oder abzukühlende Stoffstrom über die Vermittlung eines oder mehrerer Wärmeträgermedien den kolonneninternen Strom. Als Wärmeträgermedien kommen Gase, Dämpfe oder Flüssigkeiten in Frage, bevorzugt dampfförmige oder flüssige technische Wärmeträgermedien wie beispielsweise Wasser, Wärmeträger auf Mineralölbasis oder synthetische Wärmeträger (z.B. Diphyl™, Marlotherm^{®}). Besonders bevorzugte Wärmeträgermedien sind Wasser oder Wasserdampf.

Gegenstand der vorliegenden Erfindung ist daher ein Verfahren zur Reinigung von Dialkylcarbonaten in wenigstens einer Destillationskolonne enthaltend wenigstens einen Verstärkungsteil im oberen Teil der Kolonne und wenigstens einen Abtriebsteil im unteren Teil der Kolonne, dadurch gekennzeichnet, dass am Kopf einer Umesterungskolonne ein Dialkylcarbonat-Alkylalkohol-Gemisch entnommen und der Destillationskolonne zugeführt wird, wobei in der Destillationskolonne zur Aufarbeitung des Dialkylcarbonat-Alkylalkohol-Gemisches eine technische Vorrichtung zur Erhitzung des kolonneninternen Flüssigkeitstromes eingesetzt wird, wobei der kolonneninterne Flüssigkeitsstrom auf ein Temperaturniveau Tz erhitzt wird, Tz < T_{SV} ist und T_{SV} das Temperaturniveau ist, welches zum Betrieb eines Sumpfverdampfers benötigt wird und zur Erhitzung des kolonneninternen Flüssigkeitsstromes Energie teilweise oder ganz aus einem anderen chemischen Herstellverfahren gewonnen wird. Diese technische Vorrichtung wird bevorzugt im Abtriebsteil, besonders günstig in der oberen Hälfte des Abtriebsteils der Kolonne platziert.

In der Dialkylcarbonat-Aufreinigungskolonne lässt sich der Verbrauch an Wärmeenergie auf dem Temperaturniveau von T_{SV} im Sumpfverdampfer durch eine Zwischenerhitzung im Abtriebsteil, besonders günstig in der oberen Hälfte des Abtriebteils der Kolonne einsparen. Die erforderliche Menge an Wärmeenergie auf dem Temperaturniveau Tz für den Zwischenerhitzer kann aus einem anderen chemischen Herstellverfahren gewonnen werden. Dabei handelt es sich vorzugsweise um die nachfolgende Herstellung von Diarylcarbonat, wobei die erforderliche Menge an Wärmeenergie bevorzugt aus der anfallenden Kondensationswärme bei der Zwischenkondensation innerhalb der ersten Reaktionskolonne der Diarylcarbonatherstellung und / oder bei der Kondensation am Kopf der weiteren Reaktions- und / oder weiteren Destillationskolonnen der Diarylcarbonatherstellung sowie dem Kondensatsystem gewonnen werden kann.

Durch die Verringerung des Verbrauchs an Wärmeenergie mit dem Temperaturniveau Tₛᵥ unter gleichzeitiger Beibehaltung der hohen Produktqualität ergibt sich durch das erfindungsgemäße Verfahren ein signifikanter ökonomischer Vorteil.

Im Rahmen der Erfindung gereinigte Dialkylcarbonate sind bevorzugt solche der allgemeinen Formel (I) 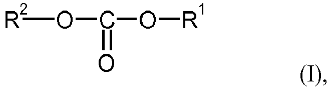 wobei R¹ und R² unabhängig voneinander für lineares oder verzweigtes, gegebenenfalls substituiertes C₁-C₃₄-Alkyl, bevorzugt für C₁-C₆-Alkyl, besonders bevorzugt für C₁-C₄-Alkyl stehen. Dabei können R¹ und R² gleich oder verschieden sein. Bevorzugt sind R¹ und R² gleich.

**C₁-C₄-Alkyl** steht im Rahmen der Erfindung beispielsweise für Methyl, Ethyl, n-Propyl, iso-Propyl, n-Butyl, sec.-Butyl, tert.-Butyl, **C₁-C₆-Alkyl** darüber hinaus beispielsweise für n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl, **C₁-C₃₄-Alkyl** darüber hinaus beispielsweise für n-Heptyl und n-Octyl, Pinakyl, Adamantyl, die isomeren Menthyle, n-Nonyl, n-Decyl, n-Dodecyl, n-Tridecyl, n-Tetradecyl, n-Hexadecyl oder n-Octadecyl. Gleiches gilt für den entsprechenden Alkylrest beispielsweise in Aralkyl- bzw. Alkylarylresten. Alkylenreste in den entsprechenden Hydroxyalkyl-oder Aralkyl- bzw. Alkylarylresten stehen beispielsweise für die den vorangehenden Alkylresten entsprechenden Alkylenreste.

Die vorangehenden Aufzählungen sind beispielhaft und nicht als Limitierung zu verstehen.

Bevorzugte Dialkylcarbonate sind Dimethylcarbonat, Diethylcarbonat, Di(n-propyl)carbonat, Di(iso-propyl)carbonat, Di(n-butyl)carbonat, Di(sec-butyl)carbonat, Di(tert-butyl)carbonat oder Dihexylcarbonat. Besonders bevorzugt sind Dimethylcarbonat oder Diethylcarbonat. Ganz besonders bevorzugt ist Dimethylcarbonat.

Die Dialkylcarbonate werden bevorzugt aus zyklischen Alkylencarbonaten mit der Formel (II) hergestellt: 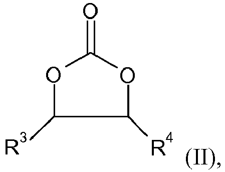 wobei in der Formel R³ und R⁴ unabhängig voneinander Wasserstoff, substituiertes oder nicht substituiertes **C₁-C₄-Alkyl,** substituiertes oder nicht substituiertes **C₂-C₄-Alkenyl** oder substituiertes oder nicht substituiertes **C₆-C₁₂-Aryl** und R³ und R⁴ gemeinsam mit den beiden Dreiring-C-Atomen einen gesättigten carbozyklischen Ring mit 5-8 Ringgliedern bedeuten können.

Die zyklischen Alkylencarbonate werden mit Alkoholen der Form

R⁵-OH

umgesetzt, wobei R⁵ ein geradkettiges oder verzweigtes **C₁**-**C₄**-**Alkyl** bedeutet.

Zur Erzeugung der Dialkylcarbonate verwendete Umesterungskatalysatoren sind die dem Fachmann bekannt, beispielsweise Hydride, Oxide, Hydroxide, Alkoholate, Amide oder Salze von Alkalimetallen, wie Lithium, Natrium, Kalium, Rubidium und Cäsium, bevorzugt von Lithium, Natrium und Kalium, besonders bevorzugt von Natrium und Kalium (US 3,642,858 A, US 3 803 201 A, EP 1 082 A). Für den Fall des Einsatzes der Alkoholate können diese auch in situ durch Einsatz der elementaren Alkalimetalle und dem umzusetzenden Alkohol gebildet werden. Salze der Alkalimetalle können solche von organischen oder anorganischen Säuren sein, wie von Essigsäure, Propionsäure, Buttersäure, Benzoesäure, Stearinsäure, Kohlensäure (Carbonate oder Hydrogencarbonate), von Salzsäure, Bromwasserstoff- oder lodwasserstoffsäure, Salpetersäure, Schwefelsäure, Fluorwasserstoffsäure, Phosphorsäure, Blausäure, Rhodanwasserstoff, Borsäure, Zinnsäure, C₁-C₄-Stannonsäuren oder Antimonsäuren. In bevorzugter Weise kommen als Verbindungen der Alkalimetalle die Oxide, Hydroxide, Alkoholate, Acetate, Propionate, Benzoate, Carbonate und Hydrogencarbonate in Frage, in besonders bevorzugter Weise werden Hydroxide, Alkoholate, Acetate, Benzoate oder Carbonate eingesetzt. Solche Alkalimetallverbindungen (gegebenenfalls in situ gebildet aus den freien Alkalimetallen) werden in Mengen von 0,001 bis 2 Gew.-%, bevorzugt 0,003 bis 1,0 Gew.-%, besonders bevorzugt 0,005 bis 1,0 Gew.-%, bezogen auf das umzusetzende Reaktionsgemisch, eingesetzt.

Es ist möglich, solchen Alkalimetallverbindungen gegebenenfalls komplexierende Stoffe zuzusetzen. Beispielsweise seien genannt Kronenether wie Dibenzo-18-krone-6, Polyethylenglykole oder bicyclische stickstoffhaltige Kryptanden.

Solche Komplexiermittel werden in Mengen von 0,1 bis 200 mol-%, bevorzugt in 1 bis 100 mol-%, bezogen auf die Alkalimetallverbindung, eingesetzt.

Als Katalysatoren für die Herstellung von Dialkylcarbonate sind ferner Thallium-I- und Thallium-lll-Verbindungen geeignet, wie die Oxide, Hydroxide, Carbonate, Acetate, Bromide, Chloride, Fluoride, Formiate, Nitrate, Cyanate, Stearate, Naphthenate, Benzoate, Cyclohexylphosphonate, Hexahydrobenzoate, das Cyclopentadienylthallium, Thalliummethylat, Thalliumethylat, bevorzugt TI-(I)-oxid, TI-(I)-hydroxid, TI-(I)-carbonat, TI-(I)-acetat, TI-(111)-acetat, TI-(I)-fluorid, TI-(I)-formiat, TI-(I)-nitrat, TI-(I)-naphtenat und TI-(I)-methylat (EP 1 083). Die Mengen an Thalliumkatalysator sind nicht besonders kritisch. Sie betragen im allgemeinen 0,0001-10 Gew.-%, bevorzugt 0,001-1 Gew.-%, bezogen auf das gesamte Reaktionsgemisch. Im Herstellungsverfahren können weiterhin stickstoffhaltige Basen als Katalysatoren eingesetzt werden (US 4 062 884). Genannt seien beispielsweise sek. oder tert. Amine wie Triethylamin, Tributylamin, Methyldibenzylamin, Dimethylcyclohexylamin u.a..

Die eingesetzten Mengen der stickstoffhaltigen Basen liegen von 0,01 bis 10 Gew.-%, bevorzugt von 0,1 bis 5 Gew.-%, besonders bevorzugt von 0,1 bis 1 Gew.-%, bezogen auf das gesamte Reaktionsgemisch. Als Katalysatoren sind ferner Verbindungen aus der Gruppe der Phosphine, Stibine, Arsine oder der zweiwertigen Schwefel- und Selen-Verbindungen sowie deren Oniumsalze einsetzbar (EP 180 387, US 4 734 519).

Beispielhaft seien die folgenden genannt: Tributylphosphin, Triphenylphosphin, Diphenylphosphin, 1,3-Bis-(diphenylphosphino)propane, Triphenylarsin, Trimethylarsin, Tributylarsin, 1,2-Bis-(diphenylarsino)ethan, Triphenylantimon, Diphenylsulfid, Diphenyldisulfid, Diphenylselenid, Tetraphenylphosphoniumhalogenid (Cl, Br, J), Tetraphenylarsoniumhalogenid (Cl, Br, J), Triphenylsulfoniumhalogenid (Cl, Br) usw.

Die bevorzugten Einsatzmengen dieser Katalysatorengruppe liegen im Bereich von 0,1 bis 10 Gew.-%, bevorzugt von 0,1 bis 5 Gew.-%, besonders bevorzugt im Bereich von 0,1 bis 2 Gew.-%, bezogen auf das gesamte Reaktionsgemisch.

Weiterhin als Katalysatoren einsetzbar sind Verbindungen des Zinns, Titans oder Zirkoniums (US-4,661,609 A). Beispiele solcher Systeme sind Butylstannonsäure, Zinnmethoxid, Dimethylzinn, Dibutylzinnoxid, Dibutylzinndilaurat, Tributylzinnhydrid, Tributylzinnchlorid, Zinn(II)ethyl-hexanoate, Zirkoniumalkoxide (Methyl, Ethyl, Butyl), Zirkonium(IV)halogenide (F, Cl, Br, J), Zirkoniumnitrate, Zirkoniumacetylacetonat, Titanalkoxide (Methyl, Ethyl, Isopropyl), Titanacetat, Titanacetylacetonat usw.

Die bevorzugt einsetzbaren Mengen dieser Katalysatoren betragen 0,1 bis 10 Gew.-%, bevorzugt 0,1 bis 5 Gew.-%, bezogen auf das Gesamtgemisch.

Im Herstellungsverfahren können weiterhin bifunktionelle Katalysatoren der Formel (III)

[AₐX_{b}]ₘ•[B_{c}Y_{d}]ₙ (III)

eingesetzt werden. In diesen bifunktionellen Katalysatoren wird das molare Verhältnis der beiden in eckigen Klammern stehenden Komponenten durch die Indices m und n ausgedrückt. Diese Indices können unabhängig voneinander Werte von 0,001-1, bevorzugt 0,01-1, besonders bevorzugt 0,05-1 und ganz besonders bevorzugt 0,1-1 annehmen. Innerhalb der eckigen Klammern stehen neutrale Salze aus je einem Kation und einem Anion. Die Indices a und b stellen unabhängig voneinander ganze Zahlen von 1-5 dar; die Indices c und d bedeuten unabhängig voneinander ganze Zahlen von 1-3, wobei den Forderungen der Valenzen der Kationen und Anionen zur Bildung solcher neutraler Salze zu entsprechen ist. Des weiteren bedeuten in (III) A das Kation eines Metalles, das der dritten Periode und Gruppe IIa, der vierten Periode und Gruppe IIa, IVa-VIIIa, Ib oder IIb, der fünften Periode und Gruppe IIa, IVa-VIIa oder IVb bzw. der sechsten Periode und Gruppe IIa-VIa des Periodensystems der Elemente in der Kurzperiodenform angehört.

Die in Frage kommenden Metalle für das Kation A entnimmt der Fachmann den üblichen Darstellungen des Periodensystems der Elemente (Mendelejew) in der Kurzperiodenform. In bevorzugter Weise handelt es sich bei A um das Kation eines der Metalle Mg, Ca, Sr, Ba, Zn, Cu, Mn, Co, Ni, Fe, Cr, Mo, W, Ti, Zr, Sn, Hf, V und Ta, in bevorzugter Weise um das Kation eines der Metalle Mg, Ca, Zn, Co, Ni, Mn, Cu und Sn. Außer den nicht komplexierten Kationen der genannten Metalle kommen auch kationische Oxokomplexe der genannten Metalle in Frage, wie beispielsweise Titanyl TiO⁺⁺ und Chromyl CrO₂⁺⁺.

Das zum Kation A gehörige Anion X ist das einer anorganischen oder organischen Säure. Eine solche anorganische oder organische Säure kann einbasisch oder zweibasisch oder dreibasisch sein. Solche Säuren und ihre Anionen sind dem Fachmann bekannt. Beispiele für Anionen einbasischer anorganischer oder organischer Säuren sind: Fluorid, Bromid, Chlorid, lodid, Nitrat, das Anion einer Alkancarbonsäure mit 1-18 C-Atomen und Benzoat; Beispiele für Anionen zweibasischer anorganischer oder organischer Säuren sind: Sulfat, Oxalat, Succinat, Fumarat, Maleinat, Phthalat und weitere; Beispiele für dreibasische anorganische oder organische Anionen sind: Phosphat oder Citrat. Bevorzugte Anionen X im Katalysator der Formel (III) sind: Fluorid, Chlorid, Bromid, lodid, Sulfat, Nitrat, Phosphat, Formiat, Acetat, Propionat, Oxalat, Butyrat, Citrat, Succinat, Fumarat, Maleinat, Benzoat, Phthalat, Decanoat, Stearat, Palmitat, und Laurinat. Besonders bevorzugte Anionen X sind: Chlorid, Bromid, lodid, Acetat, Laurinat, Stearat, Palmitat, Decanoat, Nitrat und Sulfat.

Als Kation B in den Katalysatoren der Formel (III) kommt eines aus der Gruppe der Alkali- oder Erdalkalikationen, der quaternären Ammonium-, Phosphonium-, Arsonium- oder Stibonium-Kationen und der ternären Sulfonium-Kationen in Frage.

Als (Erd)Alkalimetallkationen seien hierbei genannt: das Lithium-, Natrium-, Kalium-, Rubidium-, Cäsium-, Magnesium-, Calcium-, Strontium- und Bariumkation, bevorzugt die genannten Alkalimetallkationen, besonders bevorzugt das Natrium- und das Kaliumkation.

In bevorzugter Weise kommen als Kationen B solche der Formel (IV) 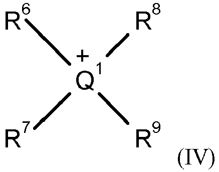 in Frage, worin
Q¹ für N, P, As oder Sb steht und
R⁶, R⁷, R⁸ und R⁹ unabhängig voneinander geradkettiges oder verzweigtes C₁-C₁₈-Alkyl- oder C₇-C₁₂-Aralkyl sind und einer der Reste R⁶-R⁹ auch C₆ -C₁₂-Aryl sein kann. In besonders bevorzugter Weise ist B ein Kation der Formel (V) 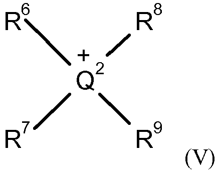 worin,
   Q² für N oder P, bevorzugt für N steht.

In ganz besonders bevorzugter Weise treten im Rahmen der Formeln (IV) bzw. (V) an die Stelle der Reste R⁶, R⁷, R⁸ und R⁹ die Reste R¹⁶, R¹⁷, R¹⁸ bzw. R¹⁹, die unabhängig voneinander geradkettiges oder verzweigtes C₁-C₁₈-Alkyl oder C₇-C₈-Aralkyl bedeuten und einer der Reste R¹⁶ bis R¹⁹ auch Phenyl sein kann. In weiterhin ganz besonders bevorzugter Weise treten an die Stelle der Reste R¹⁶, R¹⁷, R¹⁸ bzw. R¹⁹, die Reste R²⁶, R²⁷, R²⁸ bzw. R²⁹, die unabhängig voneinander C₁-C₈-Alkyl oder Benzyl bedeuten und einer der Reste R²⁶ bis R²⁹ auch Phenyl sein kann.

Geradkettiges oder verzweigtes C₁-C₁₈-Alkyl ist beispielsweise Methyl, Ethyl, Propyl, Isopropyl, Butyl, Isobutyl, Hexyl, Octyl, Dodecyl, Hexadecyl oder Octadecyl. Bevorzugtes Alkyl hat 1-12 C-Atome, besonders bevorzugtes Alkyl hat 1-8 C-Atome.

C₇-C₁₂-Aralkyl ist beispielsweise Benzyl, Phenylethyl, Phenylpropyl, Naphthylmethyl oder Naphthylethyl; bevorzugtes Aralkyl ist Benzyl oder Phenylethyl, ganz besonders bevorzugtes Aralkyl ist Benzyl.

C₆-C₁₂-Aryl ist beispielsweise Phenyl, Naphthyl oder Biphenylyl, bevorzugt Phenyl.

Das Anion Y im Katalysator der Formel (III) ist ein Halogenidion, wie Fluorid, Chlorid, Bromid oder lodid, bevorzugt Bromid oder lodid, besonders bevorzugt lodid. Es kann jedoch auch die Bedeutung von anderen unter X genannten Anionen besitzen, wenn im konkreten Fall das Anion X Bromid oder lodid ist.

Der bifunktionelle Katalysator der Formel (III) wird in einer Menge von 0,005-5 Gew.-%, bevorzugt 0,01-3 Gew.-%, besonders bevorzugt 0,01-1 Gew.-%, bezogen auf das gesamte Umesterungsgemisch, eingesetzt.

Solche Katalysatoren können homogen gelöst auf den Kopf der Kolonne gegeben werden, wobei als Lösungsmittel Alkylencarbonat, Alkylenglykol, Alkohol oder Dialkylcarbonat, also systemeigene Lösungsmittel, zur Anwendung gelangen. Es ist selbstverständlich möglich, nicht lösliche Umesterungskatalysatoren einzusetzen, die auf den Zwischenböden oder inmitten der Füllkörper angeordnet sind. In einem solchen Fall kann die Dosierung eines gelösten Katalysators über (2) entfallen. Geeignete heterogene Katalysatoren sind beispielsweise:

lonentauscherharze mit funktionellen Gruppen aus tert. Aminen, quartären Ammoniumgruppen, wobei als Gegenionen Hydroxid, Chlorid oder Hydrogensulfat beispielsweise genannt seien, Sulfonsäuregruppen oder Carboxylgruppen, wobei für beide als Gegenionen Wasserstoff, Alkalimetalle oder Erdalkalimetalle beispielhaft genannt seien. Diese funktionellen Gruppen können entweder direkt oder über inerte Ketten an das Polymer gebunden sein (US 4,062,884 A, US 4,691,041 A, EP 298 167 A). Weiterhin genannt seien Alkali- oder Erdalkalisilikate, imprägniert auf Siliciumdioxidträgern, sowie Ammonium-ausgetauschte Zeolithe.

Das Herstellungsverfahren kann kontinuierlich oder diskontinuierlich durchgeführt werden. Bevorzugt ist eine kontinuierliche Fahrweise.

Im Verfahren werden die zyklische(n) Alkylencarbonatverbindung(en) und der oder die Alkylalkohol(e) bevorzugt im Molverhältnis von 1 : 0.1 bis 1 : 40, besonders bevorzugt von 1 : 1.0 bis 1 : 30, ganz besonders bevorzugt von 1:2.0 bis 1 : 20 eingesetzt. Dabei berücksichtigt das angegebene Molverhältnis nicht die Rückführung von zyklischer Alkylencarbonatverbindung oder Alkohol in die Umesterungskolonne über einen oder mehrere Kopfkondensator(en) (vgl. unter (b)) oder einen oder mehrere etwaig vorhandene(n) Sumpfverdampfer.

Der Katalysator wird bevorzugt zusammen mit dem Strom enthaltend das zyklische Alkylencarbonat in gelöster oder suspendierter Form in die Umesterungskolonne über eine Einführungsstelle, die oberhalb der Einführungsstellen des Alkylalkohols angeordnet ist, in die Kolonne eingebracht. Alternativ kann der Katalysator auch separat beispielsweise im Alkylalkohol, im Alkylenglykol oder in einem geeigneten inerten Lösungsmittel gelöst, zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Der Umsatz von Alkylencarbonat und Alkylalkohol zu Dialkylcarbonat und Alkylenglykol findet fast vollständig in einer Umesterungskolonne statt. In bevorzugten Ausführungsformen des Verfahrens zur Herstellung von Dialkylcarbonat kann der am Sumpf dieser Umesterungskolonne entnommene Flüssigkeitsstrom - gegebenenfalls nach Aufkonzentrierung - in einem oder mehreren weiteren Schritten einer weiteren Reaktion und/oder Reinigung unterzogen werden. Bevorzugt können einzelne oder alle solche weiteren Schritte in einer oder mehreren weiteren Kolonnen erfolgen.

Als Umesterungskolonne oder gegebenenfalls zweite bzw. weitere Kolonne(n) kommen dem Fachmann bekannte Kolonnen in Frage. Dies sind beispielsweise Destillations- bzw. Rektifikationskolonnen, bevorzugt Reaktivdestillations- bzw. Reaktivrektifikationskolonnen.

Die Umesterungskolonne enthält bevorzugt wenigstens einen Verstärkungsteil im oberen Teil der Kolonne und wenigstens eine Reaktionszone unterhalb des Verstärkungsteils. Jede der zwei Sektionen weist unabhängig voneinander bevorzugt jeweils 0 bis 30, bevorzugt 0,1 bis 30 theoretische Stufen auf.

In bevorzugten Ausführungsformen weist die Umesterungskolonne unterhalb einer Reaktionszone wenigstens einen Abtriebsteil auf.

Die Umesterungskolonne kann weiterhin bevorzugt mit einem oder mehreren Sumpfverdampfer(n) ausgestattet sein. Bei Ausführung der Umesterungskolonne mit einem Abtriebsteil wird bevorzugt zusätzlich ein Sumpfverdampfer eingesetzt, welcher die aus dem Abtriebsteil ablaufende Flüssigkeit ganz oder teilweise verdampft. Dieser ganz oder teilweise verdampfte Flüssigkeitsstrom wird ganz oder teilweise wieder in die Umesterungskolonne zurückgeführt. Im Falle einer Ausführungsform ohne Abtriebsteil wird in einem gegebenenfalls eingesetzten Sumpfverdampfer die aus der Reaktionszone ablaufende Flüssigkeit ganz oder teilweise verdampft und ganz oder teilweise wieder in die Umesterungskolonne zurückgeführt.

Der oder die Verstärkungsteil(e) können in bevorzugten Ausführungsformen zusammen mit dem oder den Reaktionsteil(en) und gegebenenfalls wenigstens einem Abtriebsteil in der Umesterungskolonne untergebracht werden. Dabei wird das aus der oder den Reaktionszone(n) kommende dampfförmige Gemisch von unten in eine untere Sektion des Verstärkungsteils bzw. gegebenenfalls den unteren Verstärkungsteil geleitet, wobei eine Abreicherung des Alkylencarbonats bzw. Alkylenglykols stattfindet.

Unterhalb der Reaktionszone und eines gegebenenfalls vorhandenen Abtriebsteils wird ein Gemisch enthaltend Alkylenglykol, überschüssigem oder nicht umgesetztem Alkylencarbonat, Alkylalkohol, Dialkylcarbonat, Umesterungskatalysatoren und bei der Reaktion entstehende oder bereits in den Edukten enthaltene schwersiedende Verbindungen erhalten. Bei Verwendung eines Abtriebsteils wird der Gehalt an leichtsiedenden Verbindungen, wie beispielsweise Dialkylcarbonat und Alkohol reduziert, wobei in Anwesenheit des Umesterungskatalysators unter Umständen weiteres Dialkylcarbonat und Alkylenglykol gebildet werden. Die hierzu erforderliche Energie, wird bevorzugt durch einen oder mehrere Verdampfer zugeführt.

In allen Abschnitten der Umesterungskolonne, d.h. sowohl in Verstärkungs- und gegebenenfalls Abtriebsteil als auch in der Reaktionszone können Füllkörper oder geordnete Packungen zum Einsatz kommen. Die zu verwendenden Füllkörper bzw. geordneten Packungen sind die für Destillationen üblichen, wie sie beispielsweise in Ullmann's Encyclopädie der Technischen Chemie, 4. Aufl" Bd. 2, S. 528 ff. beschrieben sind. Als Beispiele für Füllkörper seien Raschig- oder Pall- und Novaloxringe, Berl-, Intalex- oder Torussättel, Interpackkörper und als Beispiele für geordnete Packungen seien Blech und Gewebepackungen (wie z.B. BX-Packungen, Montz Pak, Mellapak, Melladur, Kerapak und CY-Packung) aus verschiedenen Materialien, wie Glas, Steinzeug, Porzellan, Edelstahl, Kunststoff genannt. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novaloxringe, Berlsättel, BX-Packungen, Montz Pak, Mellapak, Melladur, Kerapak und CY-Packungen.

Alternativ eignen sich auch Kolonnenböden, wie beispielesweise Sieb-, Glocken-, Ventil-, Tunnelböden. In der oder den Reaktionszone(n) der Umesterungskolonne sind Kolonnenböden mit hohen Verweilzeiten bei gutem Stoffaustausch, beispielsweise Glockenböden, Ventil- oder Tunnelböden mit hohen Überlaufwehren, besonders bevorzugt. Die theoretische Bodenzahl der Reaktionszone beträgt vorzugsweise 3 bis 50, besonders bevorzugt 10 bis 50 und ganz besonders bevorzugt 10 bis 40. Der Flüssigkeits-Hold-up beträgt vorzugsweise 1 bis 80%, besonders bevorzugt 5 bis 70% und ganz besonders bevorzugt 7 bis 60% des Kolonneninnenvolumens der Reaktionszone. Die genauere Auslegung der Reaktionszone(n), des gegebenenfalls zu verwendendes Abtriebsteils und des oder der Verstärkerteil(e) kann vom Fachmann vorgenommen werden.

Die Temperatur der Reaktionszone(n) liegt bevorzugt im Bereich von 20 bis 200°C, besonders bevorzugt von 40 bis 180°C, ganz besonders bevorzugt von 50 bis 160°C. Es ist von Vorteil, die Umesterung nicht nur bei Normaldruck, sondern auch bei erhöhtem oder erniedrigtem Druck durchzuführen. Der Druck der Reaktionszone liegt daher bevorzugt im Bereich von 0,2 bis 20 bar, besonders bevorzugt von 0,3 bis 10 bar, ganz besonders bevorzugt von 0,4 bis 5 bar. Bei den vorangehend und im Folgenden aufgeführten Druckangaben handelt es sich - sofern nichts anderes explizit erwähnt - um abolute Druckangaben.

Bevorzugt wird das am Kopf der Umesterungskolonne in dem Verfahren zur Herstellung des Dialkylcarbonates entnommene Dampfgemisch enthaltend Dialkylcarbonat und Alkylalkohol nach Kondensation am Kopf der Umesterungskolonne ganz oder teilweise wenigstens einem weiteren Verfahrensschritt enthaltend wenigstens eine Destillationskolonne zur Trennung von Dialkylcarbonat und Alkylalkohol zugeführt.

Die Trennung des Dialkylcarbonats und des Alkylalkohols erfolgt bevorzugt destillativ in einer oder mehreren Destillationskolonnen oder in einer Kombination aus Destillation und Membrantrennung - im Folgenden als Hybridprozess - bezeichnet (siehe z. B. US-4,162,200 A, EP 581 115 B1, EP 592 883 B1 und WO 2007/096343A1).

Bilden Alkylalkohol und Dialkylcarbonat ein Azeotrop (z.B. Methanol und Dimethylcarbonat) so kann auch ein zweistufiges Verfahren wie beispielsweise ein Zweidruckverfahren, eine Extraktivdestillation, eine Heteroazeotropdestillation mit einem niedrigsiedenden Schleppmittel oder ein Hybridverfahren verwendet werden. Besonders bevorzugt wird das Zweidruckverfahren oder ein Hybridverfahren angewendet.

Ganz besonders bevorzugt wird die Trennung des Dialkylcarbonats und des Alkylalkohols - selbst in dem Falle, dass das Dialkylcarbonat und der Alkylalkohol ein Azeotrop bilden - in einer einzelnen Destillationskolonne durchgeführt. Diese Destillationskolonne wird bei einem Druck betrieben, der höher ist als der Druck der Umesterungskolonne(n). Der Betriebsdruck der Destillationskolonne liegt im Bereich von 1 bis 50 bar, vorzugsweise zwischen 2 und 20 bar Am Sumpf der Destillationskolonne wird das nahezu reine Dialkylcarbonat entnommen und am Kopf ein Gemisch aus Dialkylcarbonat und Alkylalkohol. Dieses Gemisch wird der oder den Umesterungskolonne(n) ganz oder teilweise zugeführt. Wird das Verfahren zur Herstellung von Dialkylcarbonat mit einem Verfahren zur Herstellung von Diarylcarbonat, welches durch Umesterung dieses Dialkylcarbonates mit einer aromatischen Hydroxyverbindung gebildet wird, gekoppelt, so kann ein Teil des Gemisches aus Dialkylcarbonat und Alkylalkohol, welches am Kopf der Destillationskolonne entnommen wird, einem entsprechenden Aufarbeitungsschritt für Alkylalkohol und Dialkylcarbonat in der Verfahrensstufe zur Herstellung von Diarylcarbonat zugeführt werden.

In einer besonders bevorzugten Ausführung, wenn das Dialkylcarbonat und der Alkylalkohol ein Azeotrop bilden, ist dieser Aufarbeitungsschritt ein Zweidruckverfahren. Solche Verfahren sind dem Fachmann grundsätzlich bekannt (vgl. z.B. Ullmann's Encyclopedia of Industrial Chemistry, Vol. 7, 2007, Kap. 6.4. und 6.5.; Chemie Ingenieur Technik (67) 11 / 95).

Bilden Alkylalkohol und Dialkylcarbonat ein Azeotrop, so weist das Destillat einer ersten Destillationskolonne des Verfahrensschritts zur Trennung von Dialkylcarbonat und Alkylalkohol vorzugsweise nahezu azeotrope Zusammensetzung auf. In diesem Fall wird dieses bevorzugt in einem Zweidruckverfahren wenigstens einer weiteren Destillationskolonne zugeführt die bei einem Betriebsdruck arbeitet, der unter dem der ersten Destillationskolonne liegt. Durch den unterschiedlichen Betriebsdruck verschiebt sich die Lage des Azeotrops hin zu geringeren Anteilen an Alkylalkohol. Man erhält als Sumpfprodukt dieser zweiten oder weiteren Destillationskolonne(n) Alkylalkohol in einer Reinheit von 90 bis 100 Gew.-%, bezogen auf das Gesamtgewicht des isolierten Sumpfproduktes, und als Destillat eine nahezu azeotropes Gemisch. Die bei geringerem Betriebsdruck arbeitende zweite oder weitere(n) Destillationskolonne(n) wird in ganz besonders bevorzugten Ausführungsformen vorzugsweise mit der Kondensationswärme des oder der Kopfkondensator(en) der ersten Destillationskolonne betrieben.

Beim Zweidruckverfahren macht man sich die Druckabhängigkeit der azeotropen Zusammensetzung eines Zweistoffgemisches zunutze. Bei einem Gemisch aus Alkylalkohol und Dialkylcarbonat, wie beispielsweise Methanol und Dimethylcarbonat, verschiebt sich die azeotrope Zusammensetzung mit zunehmendem Druck zu höheren Alkylalkoholgehalten. Führt man ein Gemisch dieser beiden Komponenten einer Kolonne (Dialkylcarbonatkolonne) zu, wobei der Alkylalkoholgehalt unterhalb der für den Betriebsdruck dieser Kolonne entsprechende azeotropen Zusammensetzung liegt, so erhält man als Destillat ein Gemisch mit nahezu azeotroper Zusammensetzung und als Sumpfprodukt nahezu reines Dialkylcarbonat. Das so erhaltene azeotrope Gemisch wird einer weiteren Destillationskolonne (Alkylalkoholkolonne) zugeführt. Diese arbeitet bei einem im Vergleich zur Dialkylcarbonatkolonne geringeren Betriebsdruck. Dadurch verschiebt sich die Lage des Azeotrops hin zu geringeren Alkylalkoholgehalten. Hierdurch ist es möglich, das in der Dialkylcarbonatkolonne erhaltene azeotrope Gemisch in ein Destillat mit nahezu azeotroper Zusammensetzung und nahezu reinen Alkylalkohol zu trennen. Das Destillat der Alkylalkoholkolonne wird wieder der Dialkylcarbonatkolonne an geeigneter Stelle zugeführt.

Der Betriebsdruck der Alkylalkoholkolonne wird vorzugsweise so gewählt, dass man diese mit der Abwärme der Dialkylcarbonatkolonne betreiben kann. Der Betriebsdruck liegt dabei zwischen 0,1 und 1 bar vorzugsweise zwischen 0,3 und 1 bar. Der Betriebsdruck der Dialkylcarbonatkolonne liegt im Bereich von 1 bis 50 bar, vorzugsweise zwischen 2 und 20 bar.

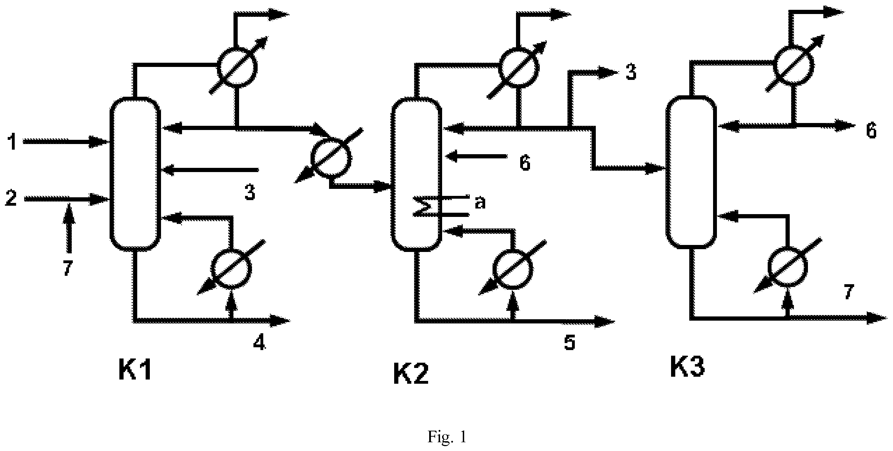

Eine beispielhafte Reaktionsführung bei der Trennung von Dialkylcarbonat und Alkylalkohol nach dem Zweidruckverfahren ist in Fig. 1 gezeigt.

Ein weiteres bevorzugtes Verfahren zur Trennung von Azeotropen aus Alkylalkohol und Dialkylcarbonat ist das Hybridverfahren. Beim Hybridverfahren erfolgt die Trennung eines Zweistoffgemisches mittels einer Kombination aus Destillation und Membranverfahren. Dabei macht man sich die Tatsache zunutze, dass man die Komponenten aufgrund ihrer polaren Eigenschaften und ihres unterschiedlichen Molekulargewichts mittels Membranen zumindest teilweise voneinander trennen kann. Im Falle eines Gemisches aus Alkylalkohol und Dialkylcarbonat, wie beispielsweise Methanol und Dimethylcarbonat, erhält man bei Verwendung geeigneter Membranen mittels Pervarporation oder Dampfpermeation ein Alkylalkohol-reiches Gemisch als Permeat und ein an Alkylalkohol verarmtes Gemisch als Retentat. Führt man ein Gemisch dieser beiden Komponenten einer Kolonne (Dialkylcarbonatkolonne) zu, wobei der Alkylalkoholgehalt unterhalb der für den Betriebsdruck dieser Kolonne entsprechenden azeotropen Zusammensetzung liegt, so erhält man als Destillat ein Gemisch mit im Vergleich zum Zulauf deutlich erhöhtem Alkylalkoholgehalt und als Sumpfprodukt nahezu reines Dialkylcarbonat.

Im Falle eines Hybridverfahrens aus Destillation und Dampfpermeation wird das Destillat der Kolonne dampfförmig entnommen. Das so erhaltende dampfförmige Gemisch wird gegebenenfalls nach Überhitzung einer Dampfpermation zugeführt. Diese wird so betrieben, dass man auf der Retentatseite nahezu den Betriebsdruck der Kolonne und auf der Permeatseite einen geringeren Druck einstellt. Der Betriebsdruck der Kolonne liegt dabei im Bereich von 1 bis 50 bar, bevorzugt zwischen 1 und 20 und besonders bevorzugt zwischen 2 und 10 bar. Der Druck auf der Permeatseite liegt zwischen 0,05 und 2 bar. Dabei erhält man auf der Permeatseite eine Alkylalkohol-reiche Fraktion mit einem Alkylalkoholgehalt von mindestens 70 Gew.-%, bevorzugt mindestens 90 Gew.-%, bezogen auf das Gesamtgewicht der Fraktion. Das Retentat, welches einen im Vergleich zum Destillat der Kolonne einen reduzierten Alkylalkoholanteil enthält, wird gegebenenfalls kondensiert und wieder der Destillationskolonne zugeführt.

Im Falle eines Hybridverfahrens aus Destillation und Pervaporation wird das Destillat der Kolonne flüssig entnommen. Das so erhaltende Gemisch wird gegebenenfalls nach Erhitzung einer Pervaporation zugeführt. Diese wird so betrieben, dass man auf der Retentatseite einen im Vergleich zur Kolonne identischen oder erhöhten Betriebsdruck auf der Permeatseite einen geringeren Druck einstellt. Der Betriebsdruck der Kolonne liegt dabei im Bereich von 1 bis 50 bar, bevorzugt zwischen 1 und 20 und besonders bevorzugt zwischen 2 und 10 bar. Der Druck auf der Permeatseite liegt zwischen 0,05 und 2 bar. Dabei erhält man auf der Permeatseite eine Alkylalkohol-reiche dampfförmige Fraktion mit einem Alkylalkoholgehalt von mindestens 70 Gew.-%, bevorzugt mindestens 90 Gew.-%, bezogen auf das Gesamtgewicht der Fraktion. Das flüssige Retentat, welches einen im Vergleich zum Destillat der Kolonne reduzierten Alkylalkoholanteil erhält, wird wieder der Destillationskolonne zugeführt. Durch die Verdampfung des Permeats wird Wärme benötigt, die gegebenenfalls nicht in ausreichendem Maße im Zulaufstrom zur Pervaporation enthalten ist. Daher kann eine Membrantrennung mittels Pervarporation gegebenenfalls mit zusätzlichen Wärmetauschern beheizt werden, wobei diese integriert oder gegebenenfalls zwischen mehreren hintereinander geschalteten Pervaporationsschritten angebracht sind.

Die Trennung von Dialkylcarbonat und Alkylalkohol erfolgt im Falle eines Hybridverfahrens besonders bevorzugt mittels einer Kombination aus Destillation und Dampfpermeation.

Die zur Trennung von Alkylalkohol und Dialkylcarbonat erforderliche Wärme wird bei einer Temperatur zwischen 100°C und 300°C, vorzugsweise zwischen 100°C und 230°C, und besonders bevorzugt zwischen 120°C und 200°C zugeführt. Um eine Wärmeintegration mit Kondensatoren der Diarylcarbonatstufe effizient zu ermöglichen, werden solche Kondensatoren der Diarylcarbonatstufe ausgewählt, in denen Dämpfe bei einer um 1°C bis 100°C, bevorzugt 2°C bis 50°C und besonders bevorzugt 5°C bis 40°C erhöhten Temperatur kondensiert werden.

In einer besonders bevorzugten Ausführungsform wird Wärmeenergie aus den Verfahrensschritten zur Herstellung von Diarylcarbonat gewonnen:
i. Zwischenkondensator der ersten Reaktionskolonne des Verfahrens zur Herstellung von Diarylcarbonat
ii. Kopfkondensator der zweiten Reaktionskolonne des Verfahrens zur Herstellung von Diarylcarbonat
iii. Kopfkondensator der Seitenstromkolonne bzw. Kondensator im Seitenstrom der ersten Destillationskolonne zur Aufreinigung des Diarylcarbonates im Verfahren zur Herstellung von Diarylcarbonat
iv. Kondensator für den Seitenstrom der zweiten Zwischensiederkolonne des Verfahrens zur Herstellung von Diarylcarbonat

Die bevorzugt mit dem oder den oben unter i.- iv. aufgeführten Kondensator(en) gewonnenene Kondensationswärme aus der Diarylcarbonatstufe kann dabei beispielsweise ganz oder teilweise mittels eines Wärmetauschers zur Erwärmung eines oder mehrerer Kolonnenabschnitte der Destillationskolonne zur Aufreinigung des Dialkylcarbonats verwendet werden. In bevorzugten Ausführungsformen wird die Kondensationswärme aus dem oder den oben unter i.- iv. aufgeführten Kondensator(en) der Diarylcarbonatstufe ganz oder teilweise mittels Zwischenerhitzer zum Erhitzen des kolonneninternen Flüssigkeitsstromes in der oder den Destillationskolonne(n) des Verfahrensschrittes zur Trennung von Dialkylcarbonat und Alkylalkohol verwendet.

Der Zwischenerhitzer kann in die Destillationskolonne integriert oder als separater Zwischenerhitzer außerhalb der Kolonne ausgeführt sein. Die Ausführung des innen- oder außenliegenden Zwischenerhitzers kann dabei sowohl 1-stufig als auch mehrstufig (d.h. ein oder mehrere Wärmetauscher) erfolgen. Für den Zwischenerhitzer sind darüber hinaus erfindungsgemäß verschiedene Konstruktionen möglich, wie z.B. integrierte Heizregister oder Heizschlangen bei innenliegender Ausführung sowie beispielsweise Plattenwärmetauscher oder Rohrbündelwärmetauscher bei außenliegender Ausführung. Solche Konstruktionen sind dem Fachmann bekannt.

In der bevorzugten innenliegenden Ausführungsform weist der Zwischenerhitzer der Destillationskolonne zur Aufreinigung des Dialkylcarbonates bevorzugt eine Länge von 100 bis 10000 mm auf und das Verhältnis von Durchmesser des Zwischenerhitzers zum Kolonnendurchmesser liegt bevorzugt bei 0,1 bis 1. Weiterhin bevorzugt weist der Zwischenerhitzer eine Wärmeübertragungsfläche von 1 bis 5000 m² auf.

Die Destillationskolonne(n) zur Aufreinigung des Dialkylcarbonats verfügen bevorzugt über einen Verstärkungsteil mit bevorzugt 5 bis 40 theoretischen Stufen zur Aufkonzentrierung des Alkylalkohols und einen Abtriebsteil mit bevorzugt 5 bis 40 theoretischen Stufen zur Aufkonzentrierung des Dialkylcarbonates.

Das Verfahren zur Herstellung von Dialkylcarbonat wird bevorzugt kontinuierlich durchgeführt.

Durch die Nutzung der Kondensationswärme aus dem oder den unter i. - iv. aufgeführten Kondensator(en) der Diarylcarbonatstufe kann die Trennung des Alkylalkohols von überschüssigem Dialkylcarbonat bei deutlich reduziertem Energieverbrauch durchgeführt werden. Die Kühlleistung in der Diarylcarbonatstufe kann dabei in gleichem Maße reduziert werden. Ein wesentlicher Vorteil des erfindungsgemäßen Verfahrens gegenüber den Verfahren gemäß Stand der Technik liegt daher in der deutlichen Reduzierung des Energieverbrauchs bei der Herstellung von Dialkylcarbonaten, Diarylcarbonaten bzw. Alkylarylcarbonaten. Gleichzeitig kann das Verfahren mit einfachem apparativem Aufwand durchgeführt werden, da aufgrund der Verwendung von Kolonnenanordnungen keine komplizierte Reaktoranordnung mit mehreren getrennten und in Serie geschalteten Reaktionszonen erforderlich ist.

Im Rahmen der Erfindung hergestellte Diarylcarbonate sind bevorzugt solche der allgemeinen Formel (VI) 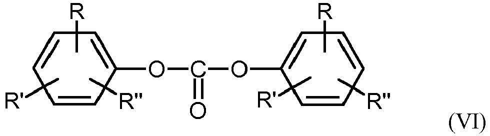 wobei R, R' und R" unabhängig voneinander H, lineares oder verzweigtes, gegebenenfalls substituiertes C₁-C₃₄-Alkyl, bevorzugt C₁-C₆-Alkyl, besonders bevorzugt C₁-C₄-Alkyl, C₁-C₃₄-Alkoxy, bevorzugt C₁-C₆-Alkoxy, besonders bevorzugt C₁-C₄-Alkoxy, C₅-C₃₄-Cycloalkyl, C₇-C₃₄-Alkylaryl, C₆-C₃₄-Aryl oder einen Halogenrest, bevorzugt einen Chlorrest darstellen und R, R' und R" auf beiden Seiten der Formel (VI) gleich oder verschieden sein können. R kann auch -COO- R'" bedeuten, wobei R'" H, gegebenenfalls verzweigtes C₁-C₃₄ Alkyl, bevorzugt C₁-C₆-Alkyl, besonders bevorzugt C₁-C₄-Alkyl, C₁-C₃₄-Alkoxy, bevorzugt C₁-C₆-Alkoxy, besonders bevorzugt C₁-C₄-Alkoxy, C₅-C₃₄-Cycloalkyl, C₇-C₃₄-Alkylaryl oder C₆-C₃₄-Aryl sein kann. Bevorzugt sind R, R' und R" auf beiden Seiten der Formel (VI) gleich. Ganz besonders bevorzugt stehen R, R' und R" für H.

Diarylcarbonate der allgemeinen Formel (VI) sind beispielsweise: Diphenylcarbonat, Methylphenylphenyl-carbonate und Di-(methylphenyl)-carbonate, auch als Gemisch, wobei die Stellung der Methylgruppe an den Phenylringen beliebig sein kann, sowie Dimethylphenyl-phenyl-carbonate und Di-(dimethylphenyl)-carbonate, auch als Gemisch, wobei die Stellung der Methylgruppen an den Phenylringen beliebig sein kann, Chlorphenyl-phenyl-carbonate und Di-(chlorphenyl)-carbonate, wobei die Stellung der Methylgruppe an den Phenylringen beliebig sein kann, 4-Ethylphenyl-phenyl-carbonat, Di-(4-ethylphenyl)-carbonat, 4-n-Propylphenyl-phenyl-carbonat, Di-(4-n-propylphenyl)-carbonat, 4-iso-Propylphenyl-phenyl-carbonat, Di-(4-iso-propylphenyl)-carbonat, 4-n-Butylphenyl-phenyl-carbonat, Di-(4-n-butylphenyl)-carbonat, 4-iso-Butylphenyl-phenylcarbonat, Di-(4-iso-butylphenyl)-carbonat, 4-tert-Butylphenyl-phenyl-carbonat, Di-(4-tert-butylphenyl)-carbonat, 4-n-Pentylphenyl-phenyl-carbonat, Di-(4-n-pentylphenyl)-carbonat, 4-n-Hexylphenyl-phenyl-carbonat, Di-(4-n-hexylphenyl)-c arb o n at, 4-iso-Octylphenyl-phenyl-carbonat, Di-(4-iso-octylphenyl)-carbonat,4-n-Nonylphenyl-phenyl-carbonat, Di-(4-n-nonylphenyl)-carbonat, 4-Cyclohexylphenylphenyl-carbonat, Di-(4-cyclohexylphenyl)-carbonat, 4-(1-Methyl-1-phenylethyl)-phenyl-phenyl-carbonat, Di-[4-(1-methyl-1-phenylethyl)-phenyl]-carbonat, Biphenyl-4-yl-phenylcarbonat, Di-(biphenyl-4-yl)-carbonat, (1-Naphthyl)-phenyl-carbonat, (2-Naphthyl)-phenyl-carbonat, Di-(1-naphthyl)-carbonat, Di-(2-naphthyl)-carbonat, 4-(1-Naphthyl)-phenyl-phenyl-carbonat, 4-(2-Naphthyl)-phenyl-phenyl-carbonat, Di-[4-(1-naphthyl)phenyl]-carbonat, Di-[4-(2-naphthyl)phenyl]-carbonat, 4-Phenoxyphenyl-phenyl-carbonat, Di-(4-phenoxyphenyl)-carbonat, 3-Pentadecylphenyl-phenyl-c arb onat, D i-(3-pentadecylphenyl)-carbonat, 4-Tritylphenyl-phenyl-carbonat, Di-(4-tritylphenyl)-c arb onat, Methylsalicylat-phenyl-carbonat, Di-(methylsalicylat)-carbonat, Ethylsalicylat-phenyl-carbonat, Di-(ethylsalicylat)-carbonat, n-Propylsalicylat-phenyl-carbonat, Di-(n-propylsalicylat)-carbonat, iso-Propylsalicylat-phenyl-carbonat, Di-(iso-propylsalicylat)-carbonat, n-Butylsalicylat-phenyl-carbonat, Di-(n-butylsalicylat)-carbonat, iso-Butylsalicylat-phenyl-carbonat, Di-(iso-butylsalicylat)-carbonat, tert-Butylsalicylat-phenyl-carbonat, Di-(tert-butylsalicylat)-carbonat, Di-(phenylsalicylat)-carbonat und Di-(benzylsalicylat)-carbonat.

Bevorzugte Diarylcarbonate sind: Diphenylcarbonat, 4-tert-Butylphenyl-phenyl-carbonat, Di-(4-tert-butylphenyl)-carbonat, Biphenyl-4-yl-phenyl-carbonat, Di-(biphenyl-4-yl)-carbonat, 4-(1-Methyl-1-phenylethyl)-phenyl-phenyl-carbonat und Di-[4-(1-methyl-1-phenylethyl)-phenyl]-carbonat.

Besonders bevorzugt ist Diphenylcarbonat.

Im Rahmen der Erfindung geeignete aromatische Hydroxyverbindungen sind bevorzugt solche der allgemeinen Formel (VII) 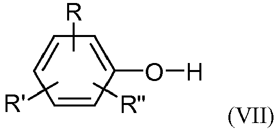 worin R, R' und R" unabhängig voneinander die für die allgemeine Formel (VI) genannte Bedeutung haben können.

Solche aromatischen Hydroxyverbindungen sind beispielsweise: Phenol, o-, m- oder p-Kresol, auch als Gemisch der Kresole, Dimethylphenol, auch als Gemisch, wobei die Stellung der Methylgruppen am Phenolring beliebig sein kann, z.B. 2,4-, 2,6-, oder 3,4-Dimethylphenol, o-, m- oder p-Chlorphenol, o-, m- oder p-Ethylphenol, o-, m- oder p-n-Propylphenol, 4-iso-Propylphenol, 4-n-Butylphenol, 4-iso-Butylphenol, 4-tert-Butylphenol, 4-n-Pentylphenol, 4-n-Hexylphenol, 4-iso-Octylphenol, 4-n-Nonylphenol, o-, m- oder p-Methoxyphenol, 4-Cyclohexylphenol,4-(1-Methyl-1-phenylethyl)-phenol, Biphenyl-4-ol, 1-Naphthol, 2-1-Naphthol, 4-(1-Naphthyl)phenol, 4-(2-Naphthyl)-phenol, 4-Phenoxyphenol, 3-Pentadecylphenol, 4-Tritylphenol, Methylsalicylsäure, Ethylsalicylsäure, n-Propylsalicylsäuret, iso-Propylsalicylsäure, n-Butylsalicylsäure, iso-Butylsalicylsäure, tert-Butylsalicylsäure, Phenylsalicylsäure und Benzylsalicylsäure.

Bevorzugte aromatischen Hydroxyverbindungen sind Phenol, 4-tert-Butylphenol, Biphenyl-4-ol und 4-(1-Methyl-1-phenylethyl)-phenol.

Besonders bevorzugt ist Phenol.

Im Rahmen der Erfindung hergestellte Alkylarylcarbonate sind bevorzugt solche der allgemeinen Formel (VIII) 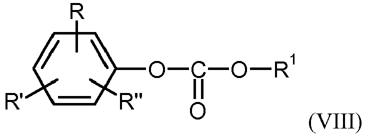 worin R, R' und R" die für die allgemeine Formel (VI) und R¹ die für die allgemeine Formel (I) genannte Bedeutung haben können.

Bevorzugte Alkylarylcarbonate sind Methyl-phenyl-carbonat, Ethyl-phenyl-carbonat, Propyl-phenyl-carbonat, Butylphenyl-carbonat und Hexyl-phenyl-carbonat, Methyl-(o-kresyl)-carbonat, Methyl-(p-kresyl)-carbonat, Ethyl-(o-kresyl)-carbonat, Ethyl-(p-kresyl)-carbonat, Methyl- oder Ethyl-(p-chlorphenyl)-carbonat. Besonders bevorzugte Alkylarylcarbonate sind Methyl-phenyl-carbonat und Ethyl-phenyl-carbonat. Ganz besonders bevorzugt ist Methyl-phenyl-carbonat.

Sowohl die für das Verfahren geeigneten Dialkylcarbonate als auch die aromatischen Hydroxyverbindungen sind dem Fachmann bekannt und kommerziell erhältlich oder können nach dem Fachmann ebenfalls bekannten Verfahren hergestellt werden.

**C₁-C₄-Alkyl** steht im Rahmen der Erfindung beispielsweise für Methyl, Ethyl, n-Propyl, iso-Propyl, n-Butyl, sec.-Butyl, tert.-Butyl, **C₁-C₆-Alkyl** darüber hinaus beispielsweise für n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl, **C₁-C₃₄-Alkyl** darüber hinaus beispielsweise für n-Heptyl und n-Octyl, Pinakyl, Adamantyl, die isomeren Menthyle, n-Nonyl, n-Decyl, n-Dodecyl, n-Tridecyl, n-Tetradecyl, n-Hexadecyl oder n-Octadecyl. Gleiches gilt für den entsprechenden Alkylrest beispielsweise in Aralkyl- bzw. Alkylarylresten. Alkylenreste in den entsprechenden Hydroxyalkyl- oder Aralkyl- bzw. Alkylarylresten stehen beispielsweise für die den vorangehenden Alkylresten entsprechenden Alkylenreste.

**Aryl** steht für einen carbocyclischen aromatischen Rest mit 6 bis 34 Gerüstkohlenstoffatomen. Gleiches gilt für den aromatischen Teil eines Arylalkylrestes, auch Aralkylrest genannt, sowie für Arylbestandteile komplexerer Gruppen, wie z.B. Arylcarbonylresten.

**Arylalkyl** bzw. **Aralkyl** bedeutet jeweils unabhängig einen geradkettigen, cyclischen, verzweigten oder unverzweigten Alkyl-Rest nach vorstehender Definition, der einfach, mehrfach oder vollständig durch Aryl-Reste gemäß vorstehender Definition substituiert sein kann.

Die vorangehenden Aufzählungen sind beispielhaft und nicht als Limitierung zu verstehen.

Es zeigt sich, dass in einer besonders bevorzugten Ausführungsform ein Verfahren zur Herstellung von wenigstens einem Diarylcarbonat aus wenigstens einem Dialkylcarbonat und wenigstens einer aromatischen Hydroxyverbindung, wobei
(a) das oder die Dialkylcarbonat(e) in Gegenwart wenigstens eines Umesterungskatalysators mit der oder den aromatischen Hydroxyverbindung(en) in einer ersten Reaktionskolonne (Diarylcarbonatherstellung) enthaltend wenigstens einen Verstärkungsteil im oberen Teil der Kolonne und wenigstens eine Reaktionszone unterhalb des Verstärkungsteils, welches mindestens zwei Sektionen aufweist, umgesetzt werden,
(b) das Sumpfprodukt der ersten Reaktionskolonne (Diarylcarbonatherstellung) wenigstens einer weiteren Reaktionskolonne enthaltend wenigstens einen Verstärkungsteil im oberen Teil der Kolonne und wenigstens eine Reaktionszone unterhalb des Verstärkungsteils zugeführt und in dieser weiter umgesetzt wird,
(c) das in den Reaktionskolonnen der Schritte (a) und/oder (b) nicht umgesetzte oder während der Reaktion gebildete Dialkylcarbonat ganz oder teilweise in wenigstens einem weiteren Verfahrensschritt enthaltend wenigstens eine Destillationskolonne vom während der Reaktion gebildeten Alkylalkohol getrennt wird,
(d) der am Kopf wenigstens einer Reaktionskolonne aus (b) gegebenenfalls nach Kondensation in wenigstens einem Kondensator entnommene aromatische Hydroxyverbindung(en) enthaltende Dampf ganz oder teilweise wenigstens einem weiteren Verfahrensschritt enthaltend wenigstens eine Destillationskolonne zugeführt wird, wobei Verbindungen, deren Siedepunkt zwischen dem des Dialkylcarbonats und dem des während der Herstellung des Diarylcarbonats gebildeten Alkylarylcarbonats liegt, abgetrennt werden,
(e) das in der oder den weiteren Reaktionskolonne(n) aus Schritt (b) erhaltene Sumpfprodukt enthaltend Diarylcarbonat wenigstens einem weiteren Verfahrensschritt zur Reinigung in wenigstens einer Destillationskolonne enthaltend wenigstens einen Verstärkungsteil im oberen Teil der Kolonne und wenigstens einen Abtriebsteil im unteren Teil der Kolonne zugeführt wird,
(f) als Sumpfprodukt aus wenigstens einer Diarylcarbonat-Destillationskolonne des Verfahrensschrittes (e) ein katalysatorhaltiger Strom erhalten wird, welcher ganz oder teilweise gegebenenfalls nach weiterer Aufreinigung wieder in das Verfahren, bevorzugt in Verfahrensschritt (a), zurückgeführt wird,
(g) aus wenigstens einer Diarylcarbonat-Destillationskolonne aus Verfahrensschritt (e) ein aromatische Hydroxyverbindundung(en) und Alkylarylcarbonat enthaltender Strom erhalten wird, welcher ganz oder teilweise wieder in das Verfahren, bevorzugt in Verfahrensschritt (a) oder (b), zurückführt wird, und
(h) aus wenigstens einer Diarylcarbonat-Destillationskolonne aus Verfahrensschritt (e) zusammen oder getrennt voneinander Verbindungen mit einem Siedepunkt oberhalb des Siedepunktes des Diarylcarbonats und Verbindungen, deren Siedepunkt zwischen dem des Dialkylcarbonats und dem des während der Herstellung des Diarylcarbonats gebildeten Alkylarylcarbonats liegt, ganz oder teilweise aus dem Verfahren ausgeschleust werden,
und wobei wenigstens eine der Reaktionskolonne(n) (Diarylcarbonatherstellung) ausgewählt aus der ersten oder der oder den weitere(n) Reaktionskolonne(n) mit einem oder mehreren Kondensatoren ausgestattet ist und die durch Kondensation in diesen Kondensatoren gewonnene Kondensationswärme direkt oder indirekt, ganz oder teilweise wieder in das Verfahren zur Herstellung von Diarylcarbonat zurückgeführt wird, sowohl eine Aufarbeitung von Produkt- und Abfallströmen als auch eine effiziente Energieintegration ermöglicht.

Die unter (h) beschriebene Ausschleusung kann vorzugsweise als flüssiger Seitenstrom aus dem Verstärkungsteil wenigstens einer Diarylcarbonat-Destillationskolonne und/oder eines Teilstrom des Destillats dieser Kolonne erfolgen.

Im Herstellungsverfahren von Diarylcarbonat werden die aromatische(n) Hydroxyverbindung(en) und das oder die Dialkylcarbonat(e) bevorzugt im Molverhältnis von 1 : 0.1 bis 1 : 10, besonders bevorzugt von 1 : 0.2 bis 1 : 5, ganz besonders bevorzugt von 1 : 0.5 bis 1 : 3 in der ersten Reaktionskolonne (Diarylcarbonatherstellung) eingesetzt. Dabei berücksichtigt das angegebene Molverhältnis nicht die Rückführung von aromatischer Hydroxyverbindung oder Dialkylcarbonat in die Reaktionskolonne über einen oder mehrere Kopfkondensator(en) (vgl. unter (b)) oder einen oder mehrere etwaig vorhandene(n) Sumpfverdampfer.

Das Verfahren zur Herstellung von Diarylcarbonaten wird in mindestens zwei Reaktionskolonnen durchgeführt.

Als erste und zweite Reaktionskolonne oder gegebenenfalls dritte bzw. weitere Kolonne(n) kommen dem Fachmann bekannte Kolonnen in Frage. Dies sind beispielsweise Destillations- bzw. Rektifikationskolonnen, bevorzugt Reaktivdestillations- bzw. Reaktivrektifikationskolonnen.

Die erste Reaktionskolonne (Diarylcarbonatherstellung) enthält wenigstens einen Verstärkungsteil im oberen Teil der Kolonne und wenigstens eine Reaktionszone unterhalb des Verstärkungsteils, welcher mindestens zwei Sektionen aufweist. Jede der zwei Sektionen weist unabhängig voneinander bevorzugt jeweils 0 bis 20, bevorzugt 0,1 bis 20 theoretische Stufen auf. In bevorzugten Ausführungsformen ist wenigstens ein Verstärkungsteil der ersten Reaktionskolonne mit wenigstens einem Zwischenkondensator ausgestattet. Der Zwischenkondensator ist vorzugsweise zwischen den beiden Sektionen des Verstärkungsteils angebracht. In diesem Fall wird der Verstärkungsteil in einen oberen und einen unteren Verstärkungsteil geteilt.

Die erste Reaktionskolonne (Diarylcarbonatherstellung) wird bevorzugt im Gegenstrom betrieben, wobei vorzugsweise in wenigstens einer Reaktionszone dieser Kolonne die aromatische Hydroxyverbindung flüssig vom Kopf zum Sumpf geführt und das Dialkylcarbonat gasförmig diesem flüssigen Strom entgegengeführt wird. Dabei wird die erste Reaktionskolonne vorzugsweise so betrieben, dass man wenigstens einer Reaktionszone, bevorzugt in das obere Drittel der Reaktionszone, einen oder mehrere, die aromatische Hydroxyverbindung und gegebenenfalls gelösten Umesterungskatalysator enthaltende Ströme, bevorzugt mit der an dieser Stelle der Kolonne herrschenden Temperatur, flüssig oder mit nur geringem Gasanteil eindosiert, wobei der Gasanteil bevorzugt weniger als 20 Gew.-% beträgt. Zudem werden einer oder mehrere, das Dialkylcarbonat enthaltende Ströme in die Reaktionszone, bevorzugt im unteren Drittel dieser Reaktionszone, gleitet, wobei die Zudosierung vorzugsweise gasförmig oder überhitzt erfolgt. In bevorzugten Ausführungsformen kann die Überhitzung des Dampfstroms 0 bis 50 °C betragen. Des Weiteren richtet sich die Taupunktstemperatur bevorzugt nach dem in der Reaktionszone an der Zudosierstelle des jeweiligen Dialkylcarbonat enthaltenden Stromes vorliegenden Druck.

Nach Passieren der Reaktionszone(n) wird der während der Reaktion gebildete Alkylalkohol, nach Durchlaufen des oder der Verstärkungsteile, am Kopf der ersten Reaktionskolonne (Diarylcarbonatherstellung) entnommen. Beim während der Reaktion gebildeten Alkylalkohol handelt es sich im Rahmen der Erfindung um den bei der Umesterung frei werdenden Alkohol, bevorzugt R¹-OH bzw. R²-OH, wobei R¹ und R² die für die allgemeine Formel (I) genannte Bedeutung haben. Der am Kopf der ersten Reaktionskolonne entnommene Strom enthält im Allgemeinen zusätzlich zum während der Reaktion gebildeten Alkylalkohol noch überschüssiges oder nicht umgesetztes Dialkylcarbonat und leichtsiedende Nebenverbindungen, wie beispielsweise Kohlendioxid oder Dialkylether. Aufgrund des oder der vorhandenen Verstärkungsteil(e) enthält dieser Strom nur geringe Mengen an höher siedenden Komponenten, wie z.B. der aromatischen Hydroxyverbindung. Der Verstärkungsteil dient zur Abtrennung der in der Reaktionszone mitverdampften höher siedenden Komponenten, wie z.B. der aromatischen Hydroxyverbindung oder Alkylarylcarbonat von den leichtsiedenden Alkylalkoholen oder Dialkylcarbonaten. Dies hat den Vorteil, dass die Trennung der während der Reaktion gebildeten Alkylalkohole von den Dialkylcarbonaten bei einem niedrigen Temperaturniveau durchgeführt werden kann.

Die erste Reaktionskolonne (Diarylcarbonatherstellung) wird in bevorzugten Ausführungsformen unter Rückflussbedingungen betrieben. Unter Rückflussbedingungen ist eine solche Fahrweise zu verstehen, bei der man den Dampfstrom am oberen Ende des Verstärkungsteils teilweise oder vollständig kondensiert (vgl. unter (b)) und das dabei anfallende Kondensat teilweise oder vollständig wieder als Rücklauf auf das obere Ende des Verstärkungsteils zurückführt. Das Rücklaufverhältnis beträgt dabei bevorzugt 0,1 bis 20, besonders bevorzugt 0,1 bis 10 und ganz besonders bevorzugt 0,1 bis 3, wobei das Rücklaufverhältnis im Rahmen der Erfindung dem Gewichtsverhältnis von in die Kolonne zurückgeführtem Kondensat zu am Kopf der Kolonne entnommenem Dampf ohne zurückgeführtes Kondensat entspricht.

In bevorzugten Ausführungsformen weist die erste Reaktionskolonne (Diarylcarbonatherstellung) unterhalb einer Reaktionszone wenigstens einen Abtriebsteil auf.

Die erste Reaktionskolonne (Diarylcarbonatherstellung) kann weiterhin bevorzugt mit einem oder mehreren Sumpfverdampfer(n) ausgestattet sein. Bei Ausführung der ersten Reaktionskolonne mit einem Abtriebsteil wird bevorzugt zusätzlich ein Sumpfverdampfer eingesetzt, welcher die aus dem Abtriebsteil ablaufende Flüssigkeit ganz oder teilweise verdampft. Dieser ganz oder teilweise verdampfte Flüssigkeitsstrom wird ganz oder teilweise wieder in die erste Reaktionskolonne zurückgeführt. Im Falle einer Ausführungsform ohne Abtriebsteil wird in einem gegebenenfalls eingesetzten Sumpfverdampfer die aus der Reaktionszone ablaufende Flüssigkeit ganz oder teilweise verdampft und ganz oder teilweise wieder in die erste Reaktionskolonne zurückgeführt.

In den bevorzugten Ausführungsformen, in denen wenigstens ein Verstärkungsteil der ersten Reaktionskolonne (Diarylcarbonatherstellung) mit wenigstens einem Zwischenkondensator ausgestattet ist, ist der Verstärkungsteil der ersten Reaktionskolonne, der mit wenigstens einem Zwischenkondensator ausgestattet ist, in einen unteren und einen oberen Verstärkungsteil (zwei Sektionen) unterteilt, wovon sich der untere Verstärkungsteil unterhalb des Zwischenkondensators und der obere Verstärkungsteil oberhalb des Zwischenkondensators befinden.

Der oder die Verstärkungsteil(e) mit wenigstens einem Zwischenkondensator können in bevorzugten Ausführungsformen zusammen mit dem oder den Reaktionsteil(en) und gegebenenfalls wenigstens einem Abtriebsteil in der Reaktionskolonne untergebracht werden. Dabei wird das aus der oder den Reaktionszone(n) kommende dampfförmige Gemisch von unten in eine untere Sektion des Verstärkungsteils bzw. gegebenenfalls den unteren Verstärkungsteil geleitet, wobei eine Abreicherung der aromatischen Hydroxyverbindung stattfindet. Das aus dieser unteren Sektion bzw. gegebenenfalls dem unteren Verstärkungsteil kommende dampfförmige Gemisch wird in einen Zwischenkondensator geleitet, wo dieses teilweise auskondensiert und das anfallende Kondensat am oberen Ende der unteren Sektion des Verstärkungsteils bzw. gegebenenfalls dem unteren Verstärkungsteil zugeführt wird.

In einer weiteren bevorzugten Ausführungsform des Verfahrens wird der Zwischenkondensator nicht in die erste Reaktionskolonne (Diarylcarbonatherstellung) integriert sondern als separater Zwischenkondensator außerhalb der ersten Reaktionskolonne ausgeführt.

In einer weiteren bevorzugten Ausführungsform des Verfahrens werden Zwischenkondensator und die obere Sektion des Verstärkungsteils nicht in die Reaktionskolonne (Diarylcarbonatherstellung) integriert sondern separat außerhalb der ersten Reaktionskolonne untergebracht.

Unterhalb der Reaktionszone und eines gegebenenfalls vorhandenen Abtriebsteils wird ein Gemisch enthaltend Alkylarylcarbonat, überschüssigem oder nichtumgesetztem Phenol, Diarylcarbonat, Umesterungskatalysatoren, Dialkylcarbonat, Alkylalkohol und bei der Reaktion entstehende oder bereits in den Edukten enthaltene schwersiedende Verbindungen erhalten. Bei Verwendung eines Abtriebsteils wird der Gehalt an leichtsiedenden Verbindungen, wie beispielsweise Dialkylcarbonat und Alkylalkohol reduziert, wobei in Anwesenheit des Umesterungskatalysators unter Umständen weiteres Alkylarylcarbonat und/oder Diarylcarbonat gebildet werden. Die hierzu erforderliche Energie, wird bevorzugt durch einen oder mehrere Verdampfer zugeführt.

In allen Abschnitten der ersten Reaktionskolonne (Diarylcarbonatherstellung), d.h. sowohl in Verstärkungs- und gegebenenfalls Abtriebsteil als auch in der Reaktionszone können Füllkörper oder geordnete Packungen zum Einsatz kommen. Die zu verwendenden Füllkörper bzw. geordneten Packungen sind die für Destillationen üblichen, wie sie beispielsweise in Ullmann's Encyclopädie der Technischen Chemie, 4. Aufl,, Bd. 2, S. 528 ff. beschrieben sind. Als Beispiele für Füllkörper seien Raschig- oder Pall- und Novaloxringe, Berl-, Intalex- oder Torussättel, Interpackkörper und als Beispiele für geordnete Packungen seien Blech und Gewebepackungen (wie z.B. BX-Packungen, Montz Pak, Mellapak, Melladur, Kerapak und CY-Packung) aus verschiedenen Materialien, wie Glas, Steinzeug, Porzellan, Edelstahl, Kunststoff genannt. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novaloxringe, Berlsättel, BX-Packungen, Montz Pak, Mellapak, Melladur, Kerapak und CY-Packungen.

Alternativ eignen sich auch Kolonnenböden, wie beispielesweise Sieb-, Glocken-, Ventil-, Tunnelböden. In der oder den Reaktionszone(n) der Reaktionskolonne (Diarylcarbonatherstellung) sind Kolonnenböden mit hohen Verweilzeiten bei gutem Stoffaustausch, beispielsweise Glockenböden, Ventil- oder Tunnelböden mit hohen Überlaufwehren, besonders bevorzugt. Die theoretische Bodenzahl der Reaktionszone beträgt vorzugsweise 3 bis 50, besonders bevorzugt 10 bis 50 und ganz besonders bevorzugt 10 bis 40. Der Flüssigkeits-Hold-up beträgt vorzugsweise 1 bis 80%, besonders bevorzugt 5 bis 70% und ganz besonders bevorzugt 7 bis 60% des Kolonneninnenvolumens der Reaktionszone. Die genauere Auslegung der Reaktionszone(n), des gegebenenfalls zu verwendendes Abtriebsteils und des oder der Verstärkerteil(e) kann vom Fachmann vorgenommen werden.

Die Temperatur der Reaktionszone(n) liegt bevorzugt im Bereich von 100 bis 300°C, besonders bevorzugt von 120 bis 250°C, ganz besonders bevorzugt von 150 bis 240°C. In bevorzugten Ausführungsformen wird in der Reaktionszone eine optimale Reaktionstemperatur einerseits durch die Wahl der Betriebsbedingungen und andererseits durch zusätzliche Wärmzufuhr im Bereich eines oder mehrerer Reaktionsböden eingestellt. Die Wärmezufuhr auf den Reaktionsböden kann dabei entweder durch Wärmetauscher oder über Reaktionsböden mit Möglichkeit zum Wärmeeintrag erfolgen. Es ist von Vorteil, die Umesterung nicht nur bei Normaldruck, sondern auch bei erhöhtem oder erniedrigtem Druck durchzuführen. Der Druck der Reaktionszone liegt daher bevorzugt im Bereich von 0.5 bis 20 bar (absolut), besonders bevorzugt von 0.8 bis 15 bar (absolut), ganz besonders bevorzugt von 0.9 bis 10 bar (absolut).

Für die in der ersten Reaktionskolonne (Diarylcarbonatherstellung) auftretenden Reaktionsschritte können aus der Literatur bekannte Umesterungskatalysatoren eingesetzt werden. Dies sind aus der Literatur bekannte Umesterungskatalysatoren für die Dialkylcarbonat-Phenol-Umesterung, wie z. B. AlX₃, TiX₃, UX₄, TiX₄, VOX₃, VX₅, ZnX₂, FeX₃, PbX₂ und SnX₄, worin X für Halogen-, Acetoxy-, Alkoxy- oder Aryloxyreste steht (DE-OS 2 58 412). Besonders bevorzugte einsetzbare Katalysatoren sind Metallverbindungen wie AlX₃, TiX₄, PbX₂ und SnX₄, wie beispielsweise Titantetrachlorid, Titantetramethoxid Titantetraphenoxid, Titantetraethoxid, Titantetraisopropylat, Titantetradodecylat, Zinntetraisooctylat und Aluminiumtriisopropylat. Ganz besonders bevorzugt sind Metallverbindungen TiX₄. Die genannten Metallverbindungen werden bevorzugt in Mengen von 0,001 bis 5 Gew.-%, bevorzugt von 0,005 bis 5 Gew.-% und besonders bevorzugt von 0,01 bis 5 Gew.-%, bezogen auf das Gewicht des umzusetzenden Reaktionsgemischs, eingesetzt.

Halogen bedeutet im Rahmen der Erfindung Fluor, Chlor oder Brom, bevorzugt Fluor oder Chlor, besonders bevorzugt Chlor.

Weitere einsetzbare Katalysatoren sind zinnorganische Verbindungen der allgemeinen Formel (R¹¹)₄₋ₓ-Sn(Y)ₓ, in der Y für einen Rest OCOR¹², OH oder OR steht, wobei R¹² C₁-C₁₂-Alkyl, C₆-C₁₂-Aryl oder C₇-C₁₃-Alkylaryl bedeutet, R¹¹ unabhängig von R¹² die Bedeutung von R¹² hat und x eine ganze Zahl von 1 bis 3 bedeutet, Dialkylzinnverbindungen mit 1 bis 12 C-Atomen im Alkylrest oder Bis-(trialkylzinn)verbindungen, beispielsweise Trimethylzinnacetat, Triethylzinnbenzoat, Tributylzinnacetat, Triphenylzinnacetat, Dibutylzinndiacetat, Dibutylzinndilaurat, Dioctylzinndilaurat, Dibutylzinnadipinat, Dibutyldimethoxyzinn, Dimethylzinnglykolat, Dibutyldiethoxyzinn, Triethylzinnhydroxid, Hexaethylstannoxan, Hexabutylstannoxan, Dibutylzinnoxid, Dioctylzinnoxid, Butylzinntriisooctylat, Octylzinntriisooctylat, Butylstannonsäure und Octylstannonsäure in Mengen von 0,001 bis 20 Gew: % (vgl. EP 879 A, EP 880 A, EP 39 452 A, DE-OS 34 45 555, JP 79/63023), polymere Zinnverbindungen der Formel -[-RR¹¹Sn-O-]-, in welcher R und R¹¹ unabhängig voneinander die vorangehend für R¹² genannte Bedeutung haben, beispielsweise Poly[oxy(dibutylstannylen)] Poly[oxy(dioctylstannylen)], Poly[oxy(butylphenyl-stannylen)] und Poly[oxy(diphenylstannylen)] (DE-OS 34 45 552), polymere Hydroxystannoxane der Formel -[-RSn(OH)-O-]-, beispielsweise Poly(ethylhydroxystannoxan), Poly(butylhydroxy-stannoxan), Poly-(octylhydroxystannoxan), Poly(undecylhydroxystannoxan) und Poly(dodecyl-hydroxystannoxane) in Mengen von 0,001 bis 20 Gew.-%, bevorzugt von 0,005 bis 5 Gew.-%, bezogen auf Dialkylcarbonat (DE-OS 40 06 520). Weitere einsetzbare Zinnverbindungen sind Sn(II)oxide der allgemeinen Formel

X-R₂Sn-O-R₂Sn-Y,

worin X und Y unabhängig voneinander OH, SCN, OR¹³, OCOR¹³ oder Halogen und R Alkyl, Aryl bedeuten soll, worin R¹³ die vorangehend für R¹² genannte Bedeutung hat (EP 0 338 760).

Als weitere einsetzbare Katalysatoren kommen Bleiverbindungen, gegebenenfalls zusammen mit Triorganophosphanen, einer Chelatverbindung oder einem Alkalimetallhalogenid, beispielsweise Pb(OH)₂-2PbCO₃, Pb(OCO-CH₃)₂, Pb(OCO-CH₃)₂ -2LiCl, Pb(OCO-CH₃)₂ • 2PPh3 in Mengen von 0,001 bis 1, bevorzugt von 0,005 bis 0,25 Mol pro Mol Dialkylcarbonat (JP 57/176932, JP 01/093580), sowie andere Blei(II)- und Blei(IV)-verbindungen, wie PbO, PbO₂ Mennige, Plumbite, und Plumbate (JP 01/093560), Eisen(III)acetat (JP 61/1 72 852), weiterhin Kupfersalze und/oder Metallkomplexe, beispielsweise von Alkali, Zink, Titan und Eisen (JP 89/005588).

Weiterhin sind in den Verfahren heterogene Katalysatorsysteme einsetzbar. Solche sind beispielsweise Mischoxide aus Silizium und Titan, die durch gemeinsame Hydrolyse von Silizium und Titanhalogeniden erhältlich sind (JP 54/125617) oder Titandioxide mit hoher BET-Oberfläche >20 m²/g (DE-OS 40 36 594)).

Bevorzugte Katalysatoren für das Verfahren sind die vorangehend genannten Metallverbindungen AlX₃, TiX₃, UX₄, TiX₄, VOX₃, VX₅, ZnX₂, FeX₃, PbX₂ und SnX₄. Besonders bevorzugt sind AlX₃, TiX₄, PbX₂ und SnX₄, wovon beispielhaft Titantetrachlorid, Titantetramethoxid Titantetraphenoxid, Titantetraethoxid, Titantetraisopropylat, Titantetradodecylat, Zinntetraisooctylat und Aluminiumtriisopropylat genannt seien. Ganz besonders bevorzugt sind Metallverbindungen TiX₄. Insbesondere bevorzugt sind Titantetramethoxid, Titantetraphenoxid und Titantetraethoxid.

Der Katalysator wird bevorzugt zusammen mit dem Strom enthaltend die aromatische(n) Hydroxyverbindung(en) in gelöster oder suspendierter Form in die erste Reaktionskolonne (Diarylcarbonatherstellung) eingebracht. Alternativ kann der Katalysator auch separat beispielsweise in einem dem Alkylalkohol entsprechenden Alkohol oder einem geeigneten inerten Lösungsmittel zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Die für die Reaktion in der ersten Reaktionskolonne (Diarylcarbonatherstellung) erforderliche Energie kann einerseits über innen oder außen liegende Vorrichtungen, wie z.B. Wärmetauscher, Verdampfer und/oder beheizbare Kolonnenböden, erzeugt und/oder andererseits sowohl mit dem flüssigen Strom enthaltend die aromatische(n) Hydroxyverbindung(en) als auch mit dem gasförmig eindosierten, Dialkylcarbonat enthaltenden Strom eingebracht werden. Insbesondere im Bereich der Reaktionszone(n) kann eine Zufuhr von Wärme auf diese Weise erfolgen. Vorzugsweise wird diese Wärme im Bereich der Reaktionszone(n) ganz oder teilweise mittels Verdampfer oder beheizbarer Kolonnenböden zugeführt. Besonders vorteilhaft ist es, die für die Reaktion in der ersten Reaktionskolonne erforderliche Energie wenigstens teilweise sowohl mit dem flüssigen Strom enthaltend die aromatische(n) Hydroxyverbindung(en) als auch mit dem gasförmig eindosierten, Dialkylcarbonat enthaltenden Strom in die ersten Reaktionskolonne und zusätzlich durch innen- und/oder außen liegende Wärmetauscher einzubringen.

Im Verfahren wird das Sumpfprodukt der ersten Reaktionskolonne einer zweiten Reaktionskolonne zugeführt.

Die zweite Reaktionskolonne (Diarylcarbonatherstellung) enthält wenigstens einen Verstärkungsteil im oberen Teil der Kolonne und wenigstens eine Reaktionszone unterhalb des Verstärkungsteils. Der Verstärkungsteil weist bevorzugt 1 bis 50, besonders bevorzugt 1 bis 25 theoretische Stufen auf.

In der zweiten Reaktionskolonne (Diarylcarbonatherstellung) wird das Sumpfpropdukt der ersten Reaktionskolonne (Diarylcarbonatherstellung), welches bereits gebildetes Alkylarylcarbonat und Diarylcarbonat enthält, flüssig oder als Dampf-Flüssigkeits-Gemisch bevorzugt der Reaktionszone, besonders bevorzugt dem oberen Teil der Reaktionszone, ganz besonders bevorzugt im oberen Drittel der Reaktionszone zugeführt. Dabei wird die zweite Reaktionskolonne vorzugsweise so betrieben, dass man das Alkylarylcarbonat teilweise oder vollständig, beispielsweise durch weitere Umesterung oder Disproportionierung, bevorzugt durch Disproportionierung, zum Diarylcarbonat umsetzt. Zusätzlich zum Sumpfprodukt der ersten Reaktionskolonne können einer oder mehrere, Alkylarylcarbonat enthaltende Ströme flüssig oder als Dampf-Flüssigkeitsgemisch im Bereich der Reaktionszone eindosiert werden. Solche zusätzlichen Alkylarylcarbonat enthaltenden Ströme können beispielsweise aus der weiteren Aufarbeitung stammen und so in das Verfahren zurückgeführt werden.

Am Kopf der zweiten Reaktionskolonne werden nicht umgesetzte aromatische Hydroxyverbindung, Dialkylcarbonat, Alkylalkohol, mittelsiedende Nebenverbindungen - wie beispielsweise Alkylarylether - und in geringem Maße leichtsiedende Nebenverbindungen abgetrennt. Im Rahmen der Erfindung sind unter mittelsiedenden Nebenverbindungen solche mit einem Siedepunkt unterhalb dem der aromatischen Hydroxyverbindung und oberhalb dem des Dialkylcarbonats zu verstehen. Solche mittelsiedenden Nebenverbindungen sind z.B. Alkylarylether, wie beispielsweise Anisol oder Phenetol. Die in der zweiten Reaktionskolonne abgetrennten mittelsiedenden Nebenverbindungen können in der ersten und/oder zweiten Reaktionskolonne bei der Reaktion entstehen oder bereits durch die Edukte in das Verfahren eingeschleust worden sein.

Der Verstärkungsteil der zweiten Reaktionskolonne (Diarylcarbonatherstellung) dient zur Abtrennung der in der Reaktionszone mitverdampften höher siedenden Komponenten, wie z.B. Alkylarylcarbonat.

Die zweite Reaktionskolonne (Diarylcarbonatherstellung) wird in bevorzugten Ausführungsformen unter Rückflussbedingungen betrieben. Unter Rückflussbedingungen ist eine solche Fahrweise zu verstehen, bei der man den Dampfstrom am oberen Ende des Verstärkungsteils teilweise oder vollständig kondensiert und das dabei anfallende Kondensat teilweise oder vollständig wieder als Rücklauf auf das obere Ende des Verstärkungsteils zurückführt. Das Rücklaufverhältnis beträgt dabei bevorzugt 0,1 bis 20, besonders bevorzugt 0,1 bis 10 und ganz besonders bevorzugt 0,1 bis 3, wobei das Rücklaufverhältnis im Rahmen der Erfindung dem Gewichtsverhältnis von in die Kolonne zurückgeführtem Kondensat zu am Kopf der Kolonne entnommenem Dampf ohne zurückgeführtes Kondensat entspricht.

Die zweite Reaktionskolonne (Diarylcarbonatherstellung) kann unterhalb einer Reaktionszone wenigstens einen Abtriebsteil aufweisen. In bevorzugten Ausführungsformen kann die Reaktionszone der zweiten Reaktionskolonne jedoch gleichzeitig als Abtriebsteil fungieren. Dabei wird das bei der Disproportionierung freigesetzte Dialkylcarbonat, durch Umesterung freigesetzter Alkylalkohol und nicht umgesetzte aromatische Hydroxyverbindung abgetrennt und gleichzeitig Diarylcarbonat und das im Wesentlichen durch Disproportionierung abreagierende Alkylarylcarbonat aufkonzentriert.

Die zweite Reaktionskolonne (Diarylcarbonatherstellung) kann weiterhin bevorzugt mit einem oder mehreren Sumpfverdampfer(n) ausgestattet sein.

Prinzipiell kann der Verstärkungsteil der zweiten Reaktionskolonne (Diarylcarbonatherstellung) ebenfalls mit einem oder mehreren Zwischenkondensatoren ausgestattet werden. Hierdurch wird der Verstärkungsteil, in einen unteren und einen oberen Verstärkungsteil (zwei Sektionen) unterteilt, wovon sich der untere Verstärkungsteil unterhalb des Zwischenkondensators und der obere Verstärkungsteil oberhalb des Zwischenkondensators befinden. In einer bevorzugten Ausführungsform weist wird die zweite Reaktionskolonne keinen Zwischenkondensator auf.

Die zweite Reaktionskolonne (Diarylcarbonatherstellung) ist mit einem oder mehreren Kondensatoren ausgestattet. Bevorzugt handelt es sich dabei um einen oder mehrere Kondensatoren am Kopf der zweiten Reaktionskolonne (Kopfkondensator(en)). Besonders bevorzugt wird eine Kaskade von Kopfkondensatoren verwendet.

Im Laufe der Kondensation in dem oder den Kondensator(en) am Kopf der zweiten Reaktionskolonne verarmen die Dämpfe an höher siedenden Komponenten, wie z.B. aromaticher Hydroxyverbindung. Um die anfallende Kondensationswärme im Sinne einer Wärmeintegration besonders effizient nutzen zu können, erfolgt die Kondensation daher bevorzugt mehrstufig, besonders bevorzugt mindestens zweistufig, in bevorzugten Ausführungsformen zwei- oder dreistufig.

Bei der besonders bevorzugten Ausführungsform der zwei- oder dreistufigen Kondensation wird die Kondensationswärme der ersten oder der ersten und zweiten Kondensationsstufe direkt oder indirekt zum Erwärmen eines Stoffstromes oder einer Kolonne innerhalb des Verfahrens verwendet, während die anfallende Kondensationswärme der zweiten oder dritten Kondensationsstufe durch Kühlwasser oder Luftkühlung abgeführt wird.

Die Kondensation am Kopf der zweiten Reaktionskolonne kann in weiteren bevorzugten Ausführungsformen zudem so durchgeführt werden, dass ein Teil der am Kopf der zweiten Reaktionskolonne entnommenen Dämpfe nicht kondensiert wird, um mittelsiedende Nebenverbindungen selektiv ausschleusen zu können.

Unterhalb der Reaktionszone und eines gegebenenfalls vorhandenen Abtriebsteils wird ein Gemisch enthaltend Alkylarylcarbonat, überschüssiger oder nichtumgesetzter aromatischer Hydroxyverbindung, Diarylcarbonat, Umesterungskatalysator(en), Dialkylcarbonat, Alkylalkohol und bei der Reaktion entstehende oder bereits in den Edukten enthaltene mittel- oder schwersiedende Nebenverbindungen erhalten. Im Rahmen der Erfindung sind unter schwersiedenden Nebenverbindungen solche mit einem Siedepunkt oberhalb dem der aromatischen Hydroxyverbindung zu verstehen.

In allen Abschnitten der zweiten Reaktionskolonne (Diarylcarbonatherstellung), d.h. sowohl in Verstärkungs- und gegebenenfalls Abtriebsteil als auch in der Reaktionszone können Füllkörper oder geordnete Packungen zum Einsatz kommen. Die zu verwendenden Füllkörper bzw. geordneten Packungen sind die für Destillationen üblichen, wie sie beispielsweise in Ullmann's Encyclopädie der Technischen Chemie, 4. Aufl,, Bd. 2, S. 528 ff. beschrieben sind. Als Beispiele für Füllkörper seien Raschig- oder Pall- und Novaloxringe, Berl-, Intalex- oder Torussättel, Interpackkörper und als Beispiele für geordnete Packungen seien Blech und Gewebepackungen (wie z.B. BX-Packungen, Montz Pak, Mellapak, Melladur, Kerapak und CY-Packung) aus verschiedenen Materialien, wie Glas, Steinzeug, Porzellan, Edelstahl, Kunststoff genannt. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novaloxringe, Berlsättel, BX-Packungen, Montz Pak, Mellapak, Melladur, Kerapak und CY-Packungen.

Alternativ eignen sich auch Kolonnenböden, wie beispielesweise Sieb-, Glocken-, Ventil-, Tunnelböden. In der oder den Reaktionszone(n) der zweiten Reaktionskolonne (Diarylcarbonatherstellung) sind Füllkörperschüttungen oder strukturierte Packungen besonders bevorzugt. Die theoretische Bodenzahl der Reaktionszone beträgt vorzugsweise 3 bis 50, besonders bevorzugt 10 bis 50 und ganz besonders bevorzugt 10 bis 40.

Die genauere Auslegung der Reaktionszone(n), des gegebenenfalls zu verwendendes Abtriebsteils und des oder der Verstärkerteil(e) kann vom Fachmann vorgenommen werden.

Die Temperatur der Reaktionszone(n) liegt bevorzugt im Bereich von 100 bis 300°C, besonders bevorzugt von 120 bis 250°C, ganz besonders bevorzugt von 180 bis 250°C.

In besonderen Ausführungsformen wird in der Reaktionszone eine optimale Reaktionstemperatur einerseits durch die Wahl der Betriebsbedingungen und andererseits durch zusätzliche Wärmzufuhr im Bereich eines oder mehrerer Reaktionsböden eingestellt. Die Wärmezufuhr auf den Reaktionsböden kann dabei entweder durch Wärmetauscher oder über Reaktionsböden mit Möglichkeit zum Wärmeeintrag erfolgen. Es ist von Vorteil, die Umesterung nicht nur bei Normaldruck, sondern auch bei erhöhtem oder erniedrigtem Druck, bevorzugt bei erniedrigtem Druck, durchzuführen. Der Druck der zweiten Reaktionskolonne (Diarylcarbonatherstellung) liegt daher bevorzugt im Bereich von 0.05 bis 20 bar (absolut), besonders bevorzugt von 0.1 bis 10 bar (absolut), ganz besonders bevorzugt von 0.1 bis 2 bar (absolut).

Für die in der zweiten Reaktionskolonne (Diarylcarbonatherstellung) auftretenden Reaktionsschritte können die vorangehend bereits für die Umesterung in der ersten Reaktionskolonne genannten Umesterungskatalysatoren eingesetzt werden. In einer bevorzugten Ausführungsform werden in der ersten und zweiten Reaktionskolonne identische Katalysatoren verwendet.

Der Katalysator wird bevorzugt zusammen mit dem Sumpfprodukt der ersten Reaktionskolonne (Diarylcarbonatherstellung) in gelöster oder suspendierter Form in die zweite Reaktionskolonne (Diarylcarbonatherstellung) eingebracht. Alternativ kann der Katalysator auch separat beispielsweise in einem dem Alkylalkohol entsprechenden Alkohol oder einem geeigneten inerten Lösungsmittel zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Die für die Reaktion in der zweiten Reaktionskolonne erforderliche Energie kann einerseits über innen oder außen liegende Vorrichtungen, wie z.B. Wärmetauscher, Verdampfer und/oder beheizbare Kolonnenböden, erzeugt und/oder andererseits sowohl mit dem flüssigen Strom enthaltend die aromatische(n) Hydroxyverbindung(en) eingebracht werden. Vorzugsweise wird diese Wärme im Bereich der Reaktionszone(n) ganz oder teilweise mittels Verdampfer zugeführt.

An die zweite Reaktionskolonne können sich eine oder mehrere weitere Reaktionskolonnen anschließen. Für solche weiteren Reaktionskolonnen gelten die vorangehend für die zweite Reaktionskolonne genannten Bedingungen und Parameterbereiche, wobei die Bedingungen und Parameter weiterer Reaktionskolonnen jedoch nicht identisch zu denen in der zweiten Reaktionskolonne sein müssen, sondern sich bevorzugt innerhalb des vorangehend genannten Rahmens der Bedingungen und Parameterbereiche von denen in der zweiten Reaktionskolonne unterscheiden. Vorzugsweise wird eine zur zweiten Reaktionskolonne zusätzliche Reaktionskolonne beispielsweise bei niedrigerem Druck betrieben als die zweite Reaktionskolonne; auch Rücklaufverhältnis und Sumpftemperatur können gegenüber denen in der zweiten Reaktionskolonne verändert sein. In einer bevorzugten Ausführungsform schließt sich im Verfahren an die erste Reaktionskolonne lediglich eine weitere Reaktionskolonne, d.h. die vorangehend genannte zweite Reaktionskolonne an. An die Reaktionskolonnen können sich jedoch weitere Kolonnen zur Aufreinigung und Trennung der Komponenten der entnommenen Ströme anschließen. Solche Kolonnen zur Aufreinigung und Trennung der Komponenten werden im Rahmen der Erfindung nicht als Reaktionskolonnen im Sinne der Erfindung verstanden.

Im Verfahren zur Herstellung von Diarylcarbonat fallen bei der Umesterung und/oder Disproportionierung in der ersten Reaktionskolonne (Diarylcarbonatherstellung) und/oder der oder den weiteren Reaktionskolonne(n) Ströme enthaltend während der Reaktion gebildeten Alkylalkohol sowie nicht umgesetztes oder während der Reaktion gebildetes Dialkylcarbonat an und werden vorzugsweise in einem oder mehreren Strömen im Gemisch entnommen. Dieses in den Reaktionskolonnen nicht umgesetzte oder während der Reaktion gebildete Dialkylcarbonat wird ganz oder teilweise in wenigstens einem weiteren Verfahrensschritt enthaltend wenigstens eine Destillationskolonne vom während der Reaktion gebildeten Alkylalkohol getrennt. Bevorzugt wird wenigstens ein Strom enthaltend nicht umgesetztes oder während der Reaktion gebildetes Dialkylcarbonat und während der Reaktion gebildeten Alkylalkohol am Kopf der ersten Reaktionskolonne (Diarylcarbonatherstellung) entnommen und zur Auftrennung wenigstens einem weiteren Verfahrensschritt enthaltend wenigstens eine Destillationskolonne zugeführt.

Bevorzugt wird das am Kopf der ersten Reaktionskolonne (Diarylcarbonatherstellung) entnommene Dampfgemisch enthaltend Dialkylcarbonat und während der Reaktion gebildetem Alkylalkohol nach Kondensation am Kopf der ersten Reaktionskolonne ganz oder teilweise wenigstens einem weiteren Verfahrensschritt enthaltend wenigstens eine Destillationskolonne zur Trennung von Dialkylcarbonat und Alkylalkohol - im Folgenden als Trenn-Destillationskolonne(n) bezeichnet - zugeführt. Besonders bevorzugt wird das dabei abgetrennte Dialkylcarbonat gegebenenfalls nach weiterer Aufreinigung wieder der ersten Reaktionskolonne zugeführt.

Die Trennung des Dialkylcarbonats und des Alkylalkohols erfolgt bevorzugt destillativ in einer oder mehreren Trenn-Destillationskolonnen oder in einer Kombination aus Destillation und Membrantrennung - auch Hybridprozess genannt - bezeichnet.

Die Trennung des Dialkylcarbonates und des Alkylalkohols erfolgt in einer der oben für die Dialkylcarbonatstufe beschriebenen analogen Art und Weise.Der am Kopf wenigstens einer Reaktionskolonne (Diarylcarbonatherstellung) aus (b) gegebenenfalls nach Kondensation in wenigstens einem Kondensator entnommene aromatische Hydroxyverbindung(en) enthaltende Dampf wird ganz oder teilweise wenigstens einem weiteren Verfahrensschritt enthaltend wenigstens eine Destillationskolonne zugeführt, wobei Verbindungen, deren Siedepunkt zwischen dem des Dialkylcarbonats und dem des während der Herstellung des Diarylcarbonats gebildeten Alkylarylcarbonats liegt - im Folgenden auch mittelsiedende Nebenverbindungen genannt - abgetrennt werden. Die in diesem Verfahrensschritt eingesetzte(n) Destillationskolonne(n) zur Abtrennung von Verbindungen, deren Siedepunkt zwischen dem des Dialkylcarbonats und dem des während der Herstellung des Diarylcarbonats gebildeten Alkylarylcarbonats liegt, werden im Folgenden auch als Zwischensieder-Destillationskolonnen bezeichnet.

In einer bevorzugten Ausführungsform des Verfahrens zur Herstellung von Diarylcarbonat wird der am Kopf wenigstens einer Reaktionskolonne aus (b) gegebenenfalls nach Kondensation in wenigstens einem Kondensator entnommene aromatische Hydroxyverbindung(en) enthaltende Dampf wenigstens einem weiteren Verfahrensschritt enthaltend wenigstens zwei Zwischensieder-Destillationskolonnen zugeführt wird, wobei das Sumpfprodukt der ersten Zwischensieder-Destillationskolonne einer zweiten Zwischensieder-Destillationskolonne zugeführt wird.

Bevorzugt wird (werden) die aus dem oder den Verfahrensschritt(en) zur Abtrennung von Verbindungen, deren Siedepunkt zwischen dem des Dialkylcarbonats und dem des während der Herstellung des Diarylcarbonats gebildeten Alkylarylcarbonats liegt, aus dem am Kopf wenigstens einer Reaktionskolonne aus (b) gegebenenfalls nach Kondensation in wenigstens einem Kondensator ganz oder teilweise entnommenen aromatische Hydroxyverbindung(en) enthaltenden Dampf erhaltene(n) aromatische(n) Hydroxyverbindung(en) wieder der ersten Reaktionskolonne zugeführt. Dabei wird (werden) die nach der Abtrennung erhaltene(n) aromatische(n) Hydroxyverbindung(en) vorzugsweise einer ersten und einzigen Zwischensieder-Destillationskolonne als Sumpfprodukt oder einer zweiten oder weiteren Zwischensieder-Destillationskolonne als Seitenstrom oder Sumpfprodukt entnommen.

Bevorzugt enthält dabei das am Kopf der ersten Zwischensieder-Destillationskolonne entnommene Produkt Dialkylcarbonat und wird ganz oder teilweise dem Verfahrensschritt (c) enthaltend wenigstens eine Trenn-Destillationskolonne zur Abtrennung des Alkylalkohols zugeführt.

In einer bevorzugten Ausführungsform werden in der ersten Zwischensieder-Destillationskolonne(n) Alkylalkohol, Dialkylcarbonat und ggf. ein Teil der mittelsiedenden Nebenkomponenten als Kopfprodukt abgetrennt. In diesem Falle verfügt diese bevorzugt über einen Verstärkungsteil mit 5 bis 40 theoretischen Stufen zur Aufkonzentrierung des Alkylalkohols und des Dialkylcarbonats und einen Abtriebsteil mit 5 bis 40 theoretischen Stufen zur Aufkonzentrierung der mittelsiedenden Nebenverbindungen. Der Betriebsdruck liegt vorzugsweise zwischen 0,05 und 3 bar absolut, besonders bevorzugt zwischen 0,1 und 2 bar absolut und ganz besonders bevorzugt zwischen 0,5 und 1,5 bar absolut. Das Rücklaufverhältnis beträgt vorzugsweise 0,1 bis 10, besonders bevorzugt 0,5 bis 5 und ganz besonders bevorzugt 0,5 bis 2.

Im Falle der oben genannten bevorzugten Ausführungsform der ersten Zwischensieder-Destillationskolonne wird die aromatische Hydroxyverbindung als Sumpfprodukt oder Seitenstrom, besonders bevorzugt als Seitenstrom, mittelsiedende Nebenverbindungen mit einem Siedepunkt oberhalb der aromatischen Hydroxyverbingung am Sumpf und mittelsiedende Nebenverbindungen mit einem Siedepunkt unterhalb der aromatischen Hydroxyverbindung als Destillat entnommen. Im Falle dieser bevorzugten Ausführung verfügt die Kolonne vorzugsweise über einen Verstärkungsteil mit mindestens einer Sektion und einer Trennleistung von 5 bis 40 theoretischen Stufen, einem Abtriebsteil mit bevorzugt mindestens einer, besonders bevorzugt mit mindestens 2 Sektionen mit einer Trennleistung von 5 bis 60 theoretischen Stufen. Der Betriebsdruck liegt vorzugsweise zwischen 0,05 und 3 bar absolut, besonders bevorzugt zwischen 0,1 und 2 bar absolut und ganz besonders bevorzugt zwischen 0,5 und 1,5 bar absolut. Das Rücklaufverhältnis beträgt vorzugsweise 1 bis 1000, besonders bevorzugt 10 bis 500 und ganz besonders bevorzugt 50 bis 200.

Im Falle einer besonders bevorzugten Ausführungsform der zweiten Zwischensieder-Destillationskolonne wird die aromatische Hydroxyverbindung als dampfförmiger Seitenstrom entnommen. Die bei der Kondensation des dampfförmigen Seitenstroms anfallende Wärme kann entweder zur Erzeugung eines Wärmeträgermediums oder direkt zur Beheizung anderer Verfahrensabschnitte zur Herstellung von Diarylcarbonaten genutzt werden.

Das in der oder den weiteren Reaktionskolonne(n) aus Schritt (b) erhaltene Sumpfprodukt enthaltend Diarylcarbonat wird wenigstens einem weiteren Verfahrensschritt zur Reinigung in wenigstens einer Destillationskolonne - im Folgenden auch erste Diarylcarbonat-Destillationskolonne genannt - enthaltend wenigstens einen Verstärkungsteil im oberen Teil der Kolonne und wenigstens einen Abtriebsteil im unteren Teil der Kolonne zugeführt. Bevorzugt wird dieser ersten Diarylcarbonat-Destillationskolonne ein Diarylcarbonat-haltiger Seitenstom entnommen. Weiterhin bevorzugt enthält das in der oder den weiteren Reaktionskolonne(n) aus Schritt (b) erhaltene Sumpfprodukt enthaltend Diarylcarbonat Verbindungen mit einem Siedepunkt zwischen dem des Diarylcarbonats und dem des während der Herstellung des Diarylcarbonats als Zwischenprodukt gebildeten Alkylarylcarbonats als Verunreinigung, welche in einem weiteren Seitenstrom der Diarylcarbonat-Destillationskolonne entnommen und gegebenenfalls in die oder eine der weiteren Reaktionskolonne(n) aus Schritt (b) zurückgeführt werden.

Bevorzugt enthält das in der oder den weiteren Reaktionskolonne(n) aus Schritt (b) erhaltene Sumpfprodukt - auch als Rohdiarylcarbonat bezeichnet - 10 bis 90 Gew.-%, besonders bevorzugt 20 bis 80 Gew.-% und ganz besonders bevorzugt 40 bis 80 Gew.-% Diarylcarbonat sowie 5 bis 90 Gew.-%, besonders bevorzugt 5 bis 60 Gew.-% und ganz besonders bevorzugt 5 bis 40 Gew.-% Alkylarylcarbonat, 1 bis 90 Gew.-%, besonders bevorzugt 1 bis 50 Gew.-% und ganz besonders bevorzugt 1 bis 30 Gew.-% aromatische Hydroxyverbindung, 0 bis 5 Gew.-%, besonders bevorzugt 0 bis 2 Gew.-% und ganz besonders bevorzugt 0 bis 0,5 Gew.-% schwersiedende Nebenkomponenten, 0 bis 5 Gew.-%, besonders bevorzugt 0,0001 bis 2 Gew.-% und ganz besonders bevorzugt 0,0001 bis 1 Gew.-% mittelsiedende Nebenkomponenten und 0,01 bis 10 Gew.-%, besonders bevorzugt 0,1 bis 5 Gew.-% und ganz besonders bevorzugt 1 bis 5 Gew.-% Katalysator, wobei die Summe aller vorangehend genannten Komponenten im zu reinigenden Diarylcarbonat 100 Gew.-% ergibt. Die Gew.-%-Angaben sind jeweils bezogen auf das gesamte Gewicht des zu reinigenden Rohdiarylcarbonats.

Mit dem Verfahren können vorzugsweise Diarylcarbonate mit einer Reinheit von, d.h. einem Gehalt von reinem Diarylcarbonat von, 99 bis 100 Gew.-%, besonders bevorzugt 99,5 bis 100 Gew.-% und ganz besonders bevorzugt 99,9 bis 100 Gew.-%, bezogen auf das Gesamtgewicht des aufgereinigten Diarylcarbonats erhalten werden.

Das im Seitenstrom der ersten Diarylcarbonat-Destillationskolonne entnommene Diarylcarbonat kann flüssig oder dampfförmig entnommen werden. Bevorzugt wird das im Seitenstrom der ersten Diarylcarbonat-Destillationkolonne entnommene Diarylcarbonat dampfförmig entnommen. In bevorzugten Ausführungsformen kann jedoch die flüssige Entnahme des Diarylcarbonats im Seitenstrom insbesondere aufgrund konstruktiver Gegebenheiten bevorzugt sein.

Die erste Diarylcarbonat-Destillationskolonne weist mindestens zwei Sektionen, d.h. einen Verstärkungsteil im oberen Teil der Kolonne und einen Abtriebsteil im unteren Teil der Kolonne auf. Bevorzugt kann der Verstärkungsteil der ersten Diarylcarbonat-Destillationskolonne in einen unteren und einen oberen Verstärkungsteil unterteilt sein. Weiterhin bevorzugt kann der Abtriebsteil der ersten Diarylcarbonat-Destillationskolonne in einen unteren und einen oberen Abtriebsteil unterteilt sein.

In einer bevorzugten Ausführungsform des Verfahrens zur Herstellung von Diarylcarbonat wird die Reinigung des in der oder den weiteren Reaktionskolonne(n) aus Schritt (b) erhaltene Sumpfprodukt enthaltend Diarylcarbonat in mindestens einer Diarylcarbonat-Destillationskolonne durchgeführt, welche wenigstens drei Sektionen aufweist. Bei diesen wenigstens drei Sektionen handelt es sich um wenigstens einen Verstärkungsteil und wenigstens einem Abtriebsteil, wobei der Abtriebsteil in einen unteren und einen oberen Abtriebsteil aufgeteilt ist. Besonders bevorzugt weist die erste Diarylcarbonat-Destillationskolonne mit einem Verstärkungsteil und einem Abtriebsteil, wobei der Abtriebsteil in einen unteren und einen oberen Abtriebsteil aufgeteilt ist, vier Sektionen auf, wobei auch der Verstärkungsteil in einen unteren und oberen Verstärkungsteil aufgeteilt ist.

Ingesamt weist die erste Diarylcarbonat-Destillationskolonne bevorzugt eine Gesamttrennleistung von 3 bis 160, besonders bevorzugt von 10 bis 90, ganz besonders bevorzugt von 13 bis 50 theoretische Stufen auf. Der obere Verstärkungsteil weist dabei bevorzugt eine Trennleistung von 0 bis 40, besonders bevorzugt 1 bis 20, ganz besonders bevorzugt 1 bis 10 theoretische Stufen, der untere Verstärkungsteil von bevorzugt 1 bis 40, besonders bevorzugt 5 bis 20, ganz besonders bevorzugt 5 bis 15 theoretische Stufen, der obere Abtriebsteil von bevorzugt 1 bis 40, besonders bevorzugt 2 bis 30, ganz besonders bevorzugt 5 bis 20 theoretische Stufen und der untere Abtriebsteil von bevorzugt 1 bis 40, besonders bevorzugt 2 bis 20, ganz besonders bevorzugt 2 bis 15 theoretische Stufen auf.

Die Verdampfung erfolgt vorzugsweise in einem Temperaturbereich von 100 bis 300°C, bevorzugt von 150 bis 240°C und besonders bevorzugt von 180 bis 230°C im Sumpf der Kolonne. Die Kondensation der Dämpfe am Kopf der Kolonne kann ein oder mehrstufig, bevorzugt ein- oder zweistufig, in einem Temperaturbereich von vorzugsweise 40 bis 250°C, bevorzugt 50 bis 200°C und besonders bevorzugt 60 bis 180°C erfolgen.

Die erste Diarylcarbonat-Destillationskolonne wird bevorzugt bei einem Kopfdruck von 1 bis 1000 mbar (absolut), besonders bevorzugt von 1 bis 100 mbar (absolut) und ganz besonders bevorzugt von 5 bis 50 mbar (absolut) betrieben. Das Rücklaufverhältnis beträgt dabei bevorzugt 0,1 bis 10, besonders bevorzugt 0,5 bis 5 und ganz besonders bevorzugt 0,5 bis 2.

In einer weiteren besonders bevorzugten Ausführungsform des Verfahrens wird das im Seitenstrom der ersten Diarylcarbonat-Destillationskolonne entnommene Diarylcarbonat in wenigstens einer, bevorzugt in einer zweiten Diarylcarbonat-Destillationskolonne aufgereinigt. In einer besonders bevorzugten Variante dieser bevorzugten Ausführungsform ist diese zweite Diarylcarbonat-Destillationskolonne ohne Abtriebsteil ausgeführt.

In dieser besonders bevorzugten Variante wird das Diarylcarbonat in einer ersten Diarylcarbonat-Destillationskolonne und einer zusätzlichen Seitenstromkolonne, der zweiten Diarylcarbonat-Destillationskolonne, aufgereinigt. Der dampfförmige Seitenstrom der ersten Diarylcarbonat-Destillationskolonne wird der Seitenstromkolonne, bevorzugt deren unterem Teil, zugeführt. Das flüssige Sumpfprodukt der Seitenstromkolonne wird in die erste Diarylcarbonat-Destillationskolonne wieder zurückgeführt.

Die Seitenstromkolonne weist bevorzugt mindestens eine Sektion auf. Besonders bevorzugt wird sie als reiner Verstärkungsteil betrieben und besitzt vorzugsweise eine Trennleistung von 1 bis 50, besonders bevorzugt von 2 bis 30 und ganz besonders bevorzugt von 5 bis 20 theoretischen Stufen.

Die Seitenstromkolonne wird bei einem Kopfdruck von 1 bis 1000 mbar (absolut), besonders bevorzugt von 1 bis 100 mbar (absolut) und ganz besonders bevorzugt von 5 bis 50 mbar (absolut) und vorzugsweise bei einem Rücklaufverhältnis von 0,1 bis 10, besonders bevorzugt von 0,2 bis 5 und ganz besonders bevorzugt von 0,2 bis 2 betrieben.

Die Kondensation der Dämpfe am Kopf der Seitenstromkolonne kann ein- oder mehrstufig in einem Kopfkondensator erfolgen. Sie erfolgt bevorzugt ein oder zweistufig in einem Temperaturbereich von 70 bis 250°C, besonders bevorzugt von 90 bis 230°C und ganz besonders bevorzugt von 90 bis 210°C. Die bei der Kondensation anfallende Abwärme kann bevorzugt zur Erzeugung von Heizdampf oder zur Beheizung anderer Verfahrensabschnitte, wie z.B. solchen bei der Herstellung von Diarylcarbonaten, verwendet werden. Das bei der Kondensation anfallende Kondensat wird teilweise wieder als Rücklauf auf die Seitenstromkolonne aufgegeben. Der restliche Teil des Kondensats wird als Destillat (aufgereinigtes Diarylcarbonat) entnommen. Inerte und/oder nicht kondensierte Dämpfe werden ausgeschleust.

Für den Fall der besonders bevorzugten Variante, dass die zweite Diarylcarbonat-Destillationskolonne ohne Abtriebsteil ausgeführt wird, kann der Verstärkungsteil dieser zweiten Diarylcarbonat-Destillationskolonne in die erste Diarylcarbonat-Destillationskolonne integriert sein. Dabei gelangt ein Teil der aus dem unteren Abtriebsteil der ersten Destillationskolonne kommenden Dämpfe in einen integrierten Verstärkungsteil, um den Gehalt an Hochsiedern zu reduzieren. Die am Kopf dieser integrierten Seitenstromkolonne austretenden Dämpfe werden in dem oder den externen Kondensator(en) kondensiert und teilweise als Rücklauf wieder auf den Kopf der zweiten Diarylcarbonat-Destillationskolonne zurückgeführt. Der restliche Teil des Kondensats wird als Destillat (gereinigtes Diarylcarbonat) entnommen. Nicht kondensierte Dämpfe werden ausgeschleust.

In einer weiteren besonders bevorzugten Variante der besonders bevorzugten Ausführungsform des Verfahrens zur Herstellung von Diarylcarbonat unter Verwendung einer zweiten Diarylcarbonat-Destillationskolonne wird diese zweite Diarylcarbonat-Destillationskolonne sowohl mit wenigstens einem Verstärkungsteil als auch mit wenigstens einem Abtriebsteil ausgeführt.

Die zweite Diarylcarbonat-Destillationskolonne weist sowohl einen Abtriebsteil als auch einen Verstärkungsteil auf. Der dampfförmige Seitenstrom der ersten Diarylcarbonat-Destillationskolonne kann dabei zunächst in einem ein- oder mehrstufigen Seitenstromkondensator kondensiert und anschließend der zweiten Diarylcarbonat-Destillationskolonne zugeführt werden. Die zweite Diarylcarbonat-Destillationskolonne wird bevorzugt bei einem Kopfdruck von 1 bis 1000 mbar (absolut), bevorzugt 1 bis 100 mbar (absolut) und besonders bevorzugt 5 bis 50 mbar (absolut) betrieben. Dabei ergibt sich im Sumpf eine Temperatur von 150 bis 300 °C, bevorzugt von 160 bis 240 °C und besonders bevorzugt 180 bis 230°C °C.

Die zweite Diarylcarbonat-Destillationskolonne weist vorzugsweise eine Gesamttrennleistung von 5 bis 100 Stufen, bevorzugt 10 bis 80 Stufen besonders bevorzugt 30 bis 80 Stufen, wobei der Verstärkungsteil davon eine Trennleistung von 1 bis 99, bevorzugt von 1 bis 79 und besonders bevorzugt von 2 bis 79 aufweist. Diese Kolonne wird bevorzugt bei einem Rücklaufverhältnis von 0,5 bis 20, bevorzugt 1 bis 10 und besonders bevorzugt 1 bis 5 betrieben.

Die Kondensation der Dämpfe am Kopf der zweiten Diarylcarbonat-Destillationskolonne kann ein- oder mehrstufig in einem Kopfkondensator erfolgen. Sie erfolgt bevorzugt ein oder zweistufig in einem Temperaturbereich von 70 bis 250°C, besonders bevorzugt von 90 bis 230°C und ganz besonders bevorzugt von 90 bis 210°C. Die bei der Kondensation anfallende Abwärme kann bevorzugt zur Erzeugung von Heizdampf oder zur Beheizung anderer Verfahrensabschnitte, wie z.B. solchen bei der Herstellung von Diarylcarbonaten, verwendet werden. Das bei der Kondensation anfallende Kondensat wird teilweise wieder als Rücklauf auf die zweite Diarylcarbonat-Destillationskolonne aufgegeben. Der restliche Teil des Kondensats wird als Destillat (aufgereinigtes Diarylcarbonat) entnommen. Nicht kondensierte Dämpfe werden ausgeschleust.

Die Verdampfung der aus dem Abtriebsteil der zweiten Diarylcarbonat-Destillationskolonne ablaufenden Flüssigkeit kann ebenfalls ein- oder mehrstufig in einem Verdampfer erfolgen.

Das Sumpfprodukt der zweiten Diarylcarbonat-Destillationskolonne kann anschließend ganz oder teilweise aus dem Verfahren ausgeschleust werden und/oder ganz oder teilweise wieder der ersten Diarylcarbonat-Destillationskolonne zugeführt werden.

Die vorangehend beschriebene besonders bevorzugte Ausführungsform des Verfahrens unter Verwendung einer zweiten Diarylcarbonat-Destillationskolonne eignet sich insbesondere für die Reinigung von Diarylcarbonaten mit erhöhten Anforderungen bezüglich deren Qualität. Solche erhöhten Anforderungen können beispielsweise in einem reduzierten Anteil hochsiedender Nebenkomponenten liegen, wobei deren Anteil im Diarylcarbonat um 10 bis 100 Gew.-%, bevorzugt 20 bis 90 Gew.-% und besonders bevorzugt 25 bis 80 Gew.-% gegenüber dem Verfahren mit nur einer Destillationskolonne reduziert werden kann.

In bevorzugten Ausführungsformen des erfindungsgemäßen Verfahrens kann wenigstens eine der im Verfahren eingesetzten Reaktionskolonnen und/oder wenigstens eine der im Verfahren eingesetzten Destillationskolonnen einen oder mehrere Kopfkondensatoren aufweisen, welche in die Kolonne integriert sind, wobei das Verhältnis d/D von Durchmesser der Dampfleitung von der Kolonne zu dem oder den Kopfkondensator(en) (d) zu Kolonnendurchmesser der Kolonne (D) im Bereich von 0,2 bis 1, bevorzugt im Bereich von 0,5 bis 1 liegt. In einer besonders bevorzugten Ausführungsform kann der Kopfkondensator in die Destillationskolonne integriert sein, so dass zwischen Destillationskolonne und Kopfkondensator keine Dampfleitung erforderlich ist. Das Verhältnis d/D beträgt in diesem Fall 1. Dabei kann der Kolonnenquerschnitt nach Eintritt in den Kopfkondensator unter Umständen auch dem Kondensationsfortschritt angepasst werden. Entsprechende Anordnungen sind auch für die anderen im Verfahren eingesetzten Destillationskolonnen und/oder Reaktionskolonnen möglich. Bevorzugt weisen mehrere der im Verfahren eingesetzten Reaktionskolonnen und/oder Destillationskolonnen einen der vorangehend genannten Kopfkondensatoren auf.

Bei einigen Kondensatorformen kann es von Vorteil sein, den Kolonnenquerschnitt variabel zu gestalten. Ist die Führung der zu kondensierenden Dämpfe beispielsweise von unten nach oben, so nimmt die Dampfmenge nach oben hin ab. Durch eine Reduktion des Kolonnendurchmessers in Richtung Kolonnenkopf, wird der für den Dampf verfügbare Kolonnenquerschnitt der nach oben hin abnehmenden Dampfmenge angepasst. Dabei muss die Entnahme der nicht kondensierten Dämpfe nicht zwangsläufig oben erfolgen. Wird beispielsweise eine Konstruktion gewählt, bei der ein Platten- oder Rohrbündel von oben in die Kolonne einfügt wird, so kann sich die Entnahme der nicht kondensierten Dämpfe auch seitlich befinden.

In bevorzugten Ausführungsformen des erfindungsgemäßen Verfahrens können Leitungen und Aggregate, die Gemische führen, welche einen Festpunkt von mehr als 30°C, bevorzugt mehr als 40°C aufweisen, auf Temperaturen oberhalb dieses Festpunktes, bevorzugt auf Temperaturen von mehr als 1°C oberhalb dieses Festpunktes, besonders bevorzugt auf Temperaturen von mehr als 5°C oberhalb dieses Festpunktes beheizt werden. Dadurch werden Ausfällungen von Feststoffen innerhalb dieser Leitungen und Aggregate vermieden und die Wiederinbetriebnahme der entsprechenden Anlagen nach Stillständen erheblich erleichtert.

In besonders bevorzugten Ausführungsformen des erfindungsgemäßen Verfahrens wird die Kondensationsenergie, die in einem oder mehreren Kondensator(en), ausgewählt aus der Gruppe bestehend aus
i. dem oder den gegebenenfalls vorhandenen Zwischenkondensator(en) der ersten Reaktionskolonne(n) (Diarylcarbonatherstellung),
ii. dem oder den Kondensator(en), vorzugsweise Kopfkondensator(en) der zweiten oder weiteren Reaktionskolonne(n) (Diarylcarbonatherstellung),
iii. dem oder den Kondensator(en) zur Kondensation des Seitenstroms enthaltend das aufgereinigte Diarylcarbonat oder, im Falle, dass eine zweite Diarylcarbonat-Destillationskolonne oder Seitenstromkolonne, vorhanden ist, vorzugsweise dem oder den Kopfkondensator(en) der zweiten Diarylcarbonat-Destillationskolonne oder der Seitenstromkolonne, vorzugsweise der Seitenstromkolonne und
iv. dem oder den Kondensator(en) zur Kondensation des dampfförmigen Seitenstromes der zweiten Zwischensiederkolonne (Diarylcarbonatherstellung),
gewonnen wird,
direkt oder indirekt, ganz oder teilweise in die Verfahrensstufe zur Reinigung des Dialkylcarbonates im Verfahren zur Herstellung des Dialkylcarbonates geführt und bevorzugt zur Zwischenerhitzung des kolonneninternen Flüssigkeitstromes der Destillationskolonne zur Reinigung des Dialkylkcarbonates verwendet wird.

Ganz besonders bevorzugt wird die Kondensationsenergie, die in den unter iii. und/oder iv. genannten Kondensatoren freigesetzt wird, zur Zwischenerhitzung des kolonneninternen Flüssigkeitstromes eingesetzt.

Unter direkter Rückführung der Kondensationswärme in das Verfahren ist im Rahmen der Erfindung zu verstehen, dass diese Kondensationswärme ohne zwischengeschaltetes Heizmedium in das Verfahren zurückgeführt wird, z.B. zur Erwärmung entweder eines oder mehrerer Ströme oder zur Erwärmung eines oder mehrerer Kolonnenabschnitte innerhalb des Verfahrens. Dies kann beispielsweise in einem Wärmetauscher erfolgen. Vorzugsweise ist dabei entweder ein solcher Wärmeaustauscher mit dem oder den Kondensatoren kombiniert. Unter indirekter Rückführung der Kondensationswärme in das Verfahren ist im Rahmen der Erfindung zu verstehen, dass mit der gewonnen Kondensationswärme zunächst ein Heizmedium erzeugt wird, welches zur Rückführung der Kondensationswärme in das Verfahren dient. Mit diesem Heizmedium können beispielsweise ein oder mehrere Ströme oder einer oder mehrere Kolonnenabschnitte innerhalb des Verfahrens erwärmt werden. Als Heizmedium kommen Gase, Dämpfe oder Flüssigkeiten in Frage, bevorzugt dampfförmige oder flüssige technische Wärmeträgermedien wie beispielsweise Wasser, Wärmeträger auf Mineralölbasis oder synthetische Wärmeträger (z.B. Diphyl™, Marlotherm^{®}). Besonders bevorzugte Heizmedien sind Wasser oder Wasserdampf.

Durch die Nutzung der Kondensationswärme kann die Trennung des Alkylalkohols von Dialkylcarbonat bei deutlich reduziertem Energieverbrauch durchgeführt werden. Die Kühlleistung in der Verfahrensstufe zur Herstellung von Diarylcarbonat kann dabei in gleichem Maße reduziert werden. Ein wesentlicher Vorteil des erfindungsgemäßen Verfahrens gegenüber den Verfahren gemäß Stand der Technik liegt daher in der deutlichen Reduzierung des Energieverbrauchs bei der Herstellung von Dialkylcarbonaten und Diarylcarbonaten bzw. Alkylarylcarbonaten. Gleichzeitig kann das Verfahren mit einfachem apparativem Aufwand durchgeführt werden.In den Abbildungen bedeuten:
- K1: Umesterungskolonne
- K2: erste Destillationskolonne zur Trennung des Gemisches enthaltend Dialkylcarbonat und Alkylalkohol
- K3: zweite Destillationskolonne zur Trennung des Gemisches enthaltend Dialkylcarbonat und Alkylalkohol
- 1: Eduktstrom enthaltend Alkylencarbonat und/oder optional Katalysator
- 2: Eduktstrom enthaltend nahezu reinen Alkylalkohol
- 3: Eduktstrom enthaltend Alkylalkohol und Dialkylcarbonat
- 4: Strom enthaltend Alkylenglykol
- 5: Strom enthaltend aufgereinigtes Dialkylcarbonat
- 6: Strom enthaltend Dialkylcarbonat und Alkylalkohol
- 7: Strom enthaltend nahezu reinen Alkylalkohol
- 8: Strom enthaltend Extraktionsmittel (vorzugsweise Alkylencarbonat)
- 9: Strom enthaltend Extraktionsmittel (vorzugsweise Alkylencarbonat)
- 10: Strom enthaltend Extraktionsmittel (vorzugsweise Alkylencarbonat)
- K1: erste Reaktionskolonne (Diarylcarbonatherstellung)
- K12: zweite Reaktionskolonne (Diarylcarbonatherstellung)
- K13: erste Destillationskolonne zur Reinigung von Diarylcarbonat (Diarylcarbonatherstellung)
- K14: Seitenstromkolonne zur Reinigung von Diarylcarbonat
- K17: erste Zwischensiederkolonne zur Abtrennung von Verbindungen, die einen geringeren Siedepunkt besitzen als die aromatische Hydroxyverbindung, am Kopf der Kolonne
- K18: zweite Zwischensiederkolonne zur Abtrennung von u.a. der aromatischen Hydroxyverbindung im Seitenstrom
- i.: Zwischenkondensator der ersten Reaktionskolonne (K11) des Verfahrens zur Herstellung von Diarylcarbonat
- ii.: Kopfkondensator der zweiten Reaktionskolonne (K12) des Verfahrens zur Herstellung von Diarylcarbonat
- iii.: Kopfkondensator der Seitenstromkolonne (K14) bzw. Kondensator im Seitenstrom der ersten Destillationskolonne (K13) zur Aufreinigung des Diarylcarbonates im Verfahren zur Herstellung von Diarylcarbonat
- iv.: Kondensator für den Seitenstrom der zweiten Zwischensiederkolonne (K18) des Verfahrens zur Herstellung von Diarylcarbonat

Fig. 1 beschreibt einen Umesterungsschritt von Alkylencarbonat und Alkylalkohol mittels Reaktivrektifikation in einer ersten Umesterungskolonne (K1) im Allgemeinen und die Aufarbeitung des am Kopf der Umesterungskolonne anfallenden Gemisches enthaltend Dialkylcarbonat und Alkylalkohol mittels Zweidruckdestillation in einer ersten (K2) und einer zweiten (K3) Destillationskolonne mit einem Zwischenerhitzer (a) in der ersten Destillationskolonne.

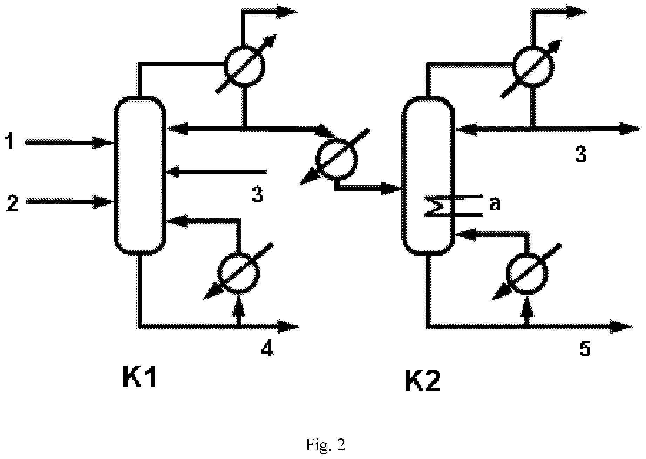

Fig. 2 beschreibt einen Umesterungsschritt von Alkylencarbonat und Alkylalkohol mittels Reaktivrektifikation in einer ersten Umesterungskolonne (K1) im Allgemeinen und die Aufarbeitung des am Kopf der Umesterungskolonne anfallenden Gemisches enthaltend Dialkylcarbonat und Alkylalkohol mittels einer einzelnen Destillationskolonne enthaltend einen Zwischenerhitzer (a).

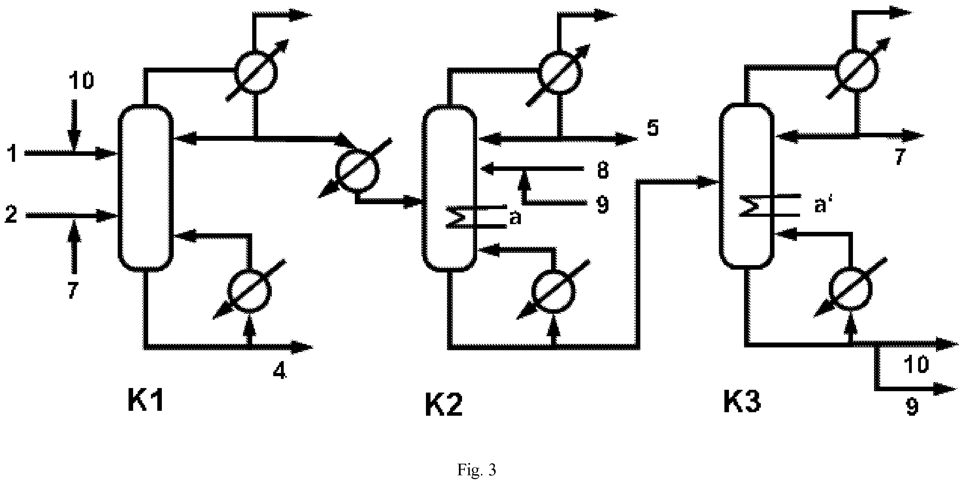

Fig. 3 beschreibt einen Umesterungsschritt von Alkylencarbonat und Alkylalkohol mittels Reaktivrektifikation in einer ersten Umesterungskolonne (K1) im Allgemeinen und die Aufarbeitung des am Kopf der Umesterungskolonne anfallenden Gemisches enthaltend Dialkylcarbonat und Alkylalkohol mittels Extraktivdestillation in einer ersten (K2) und einer zweiten (K3) Destillationskolonne mit einem Zwischenerhitzer (a) in der ersten Destillationskolonne und optional einem Zwischenerhitzer (a') in der zweiten Destillationskolonne, wobei das Alkylencarbonat vorzugsweise als Extraktionsmittel verwendet wird.

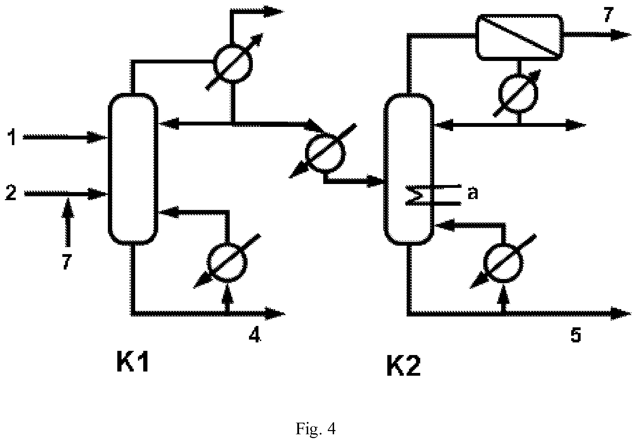

Fig. 4 beschreibt einen Umesterungsschritt von Alkylencarbonat und Alkylalkohol mittels Reaktivrektifikation in einer ersten Umesterungskolonne (K1) im Allgemeinen und die Aufarbeitung des am Kopf der Umesterungskolonne anfallenden Gemisches enthaltend Dialkylcarbonat und Alkylalkohol mittels Destillation und Dampfpermeation in einer Destillationskolonne (K2) mit einem Zwischenerhitzer (a).

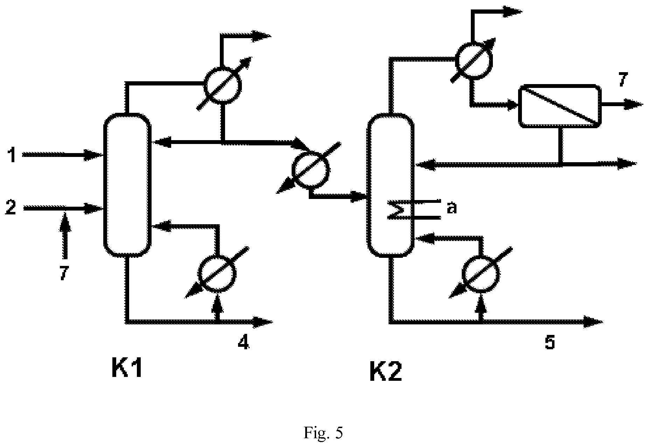

Fig. 5 beschreibt einen Umesterungsschritt von Alkylencarbonat und Alkylalkohol mittels Reaktivrektifikation in einer ersten Umesterungskolonne (K1) im Allgemeinen und die Aufarbeitung des am Kopf der Umesterungskolonne anfallenden Gemisches enthaltend Dialkylcarbonat und Alkylalkohol mittels Destillation und Pervaporation in einer Destillationskolonne (K2) mit einem Zwischenerhitzer (a).

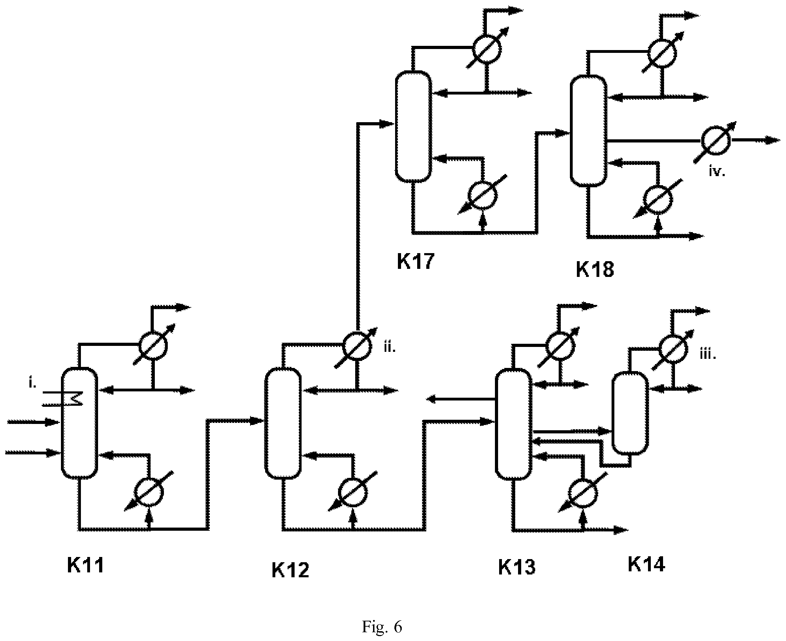

Fig. 6 beschreibt ausschnittsweise im Allgemeinen das Verfahren zur Herstellung von Diarylcarbonat aus Dialkylcarbonat und einer aromatischen Monohydroxyverbindung mittels Reaktivrektifikation in einer ersten Reaktionskolonne (K11), einer zweiten Reaktionskolonne (K12), einer Destillationskolonne (K13) zur Aufreinigung des Roh-Diarylcarbonates, einer mit dieser Destillationskolonnen verbundenen Seitenstromkolonne (K14) zur weiteren Reinigung des Diarylcarbonates, einer ersten Zwischensiederkolonne (K17) und einer zweiten Zwischensiederkolonne (K18), wobei i., ii., iii.., und iv. die Wärmetauscher bezeichnen, in denen die Kondensationsenergie gewonnen werden kann, mit der der oder die Zwischenerhitzer (a und/oder a') in den Destillationskolonnen K2 und/oder K3 der Verfahrensstufe zur Herstellung von Dialkylcarbonat betrieben wird/werden.

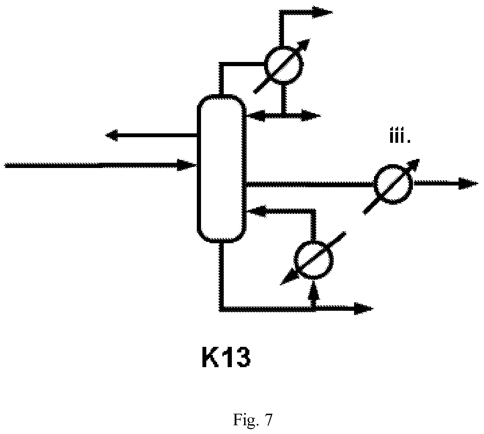

Fig 7 beschreibt die Aufreinigung des Diarylcarbonates mit einer Destillationskolonne und der Entnahme und Kondensation des Diarylcarbonates im Seitenstrom mittels des Kondensators iii., wobei die Kondensationswärme für den oder die Zwischenerhitzer (a und/oder a') in den Destillationskolonnen K2 und/oder K3 der Verfahrensstufe zur Herstellung von Dialkylcarbonat verwendet wird.

Anhand eines Beispiels wird nun die bevorzugte Betriebsweise für das erfindungsgemäße Verfahren detailliert aufgezeigt. Beispiel 1 zeigt die bevorzugte Betriebsweise für die Dialkylcarbonat-Aufreinigungskolonne. Dieses Beispiel soll auf keine Weise als Limitierung der Erfindung ausgelegt werden.

Der Vorteil dieser Erfindung, nämlich die Verringerung des Verbrauchs an Wärmeenergie auf dem Temperaturniveau Tₛᵥ, welche bevorzugt in Form von Heizdampf zur Verfügung gestellt wird, durch Installation einer technischen Vorrichtung zur Zwischenerhitzung, gegenüber anderen Betriebsarten ohne den bereits erwähnten Zwischenerhitzer, wird in den Beispielen dargestellt.

### Beispiel:

Eine Destillationskolonne zur Aufreinigung des bei der Umesterung entstehenden Dialkylcarbonats bestehend aus einem Verstärkungsteil mit 28 theoretischen Stufen und einem Abtriebsteil mit 11 theoretischen Stufen wird bei einem am Kopf der Kolonne gemessenen Druck von 10 bar (absolut) und einem Rücklaufverhältnis von 1,0 betrieben.

Im unteren Kolonnenbereich wird zwischen der 27. und 28. theoretischen Trennstufe kontinuierlich 30644 kg/h eines Dialkylcarbonat enthaltenden Alkoholgemischs mit 59 Gew.-% MeOH und 41 Gew.-% Dimethylcarbonat zudosiert.

Ein Partialkondensator kondensiert den Dampfstrom am Kopf der Kolonne bei 137 °C. Man erhält sowohl 21 kg/h dampfförmiges als auch 21378 kg/h flüssiges Destillat mit einer Zusammensetzung von 84 Gew.-% Methanol und 16 Gew.-% Dimethylcarbonat.

Auf der 28. theoretischen Stufe der Kolonne ist eine Vorrichtung zur Zwischenerhitzung mit einer Heizleistung von 6000 kW installiert, die mit Hilfe von Dampf auf einer Druckstufe von 6 bar als Heizmedium betrieben wird. Der genannte Heizdampf wird aus der nachfolgenden Prozesskette, nämlich aus der Herstellung von Diphenylcarbonat, gewonnen.

Man erhält 9245 kg/h flüssiges Sumpfprodukt mit einer Zusammensetzung von 99,5 Gew.-% Dimethylcarbonat und 0,5 Gew.-% Methanol. Der Sumpfverdampfer wird mit Heizdampf auf einer Druckstufe von 16 bar bei 183 °C betrieben und hat eine Heizleistung von 4391 kW.

### Vergleichsbeispiel:

Es wird die gleiche konventionelle Destillationskolonne, wie im Beispiel beschrieben, zur Aufreinigung des bei der Umesterung entstehenden Dialkylcarbonats verwendet. Die Kolonne wird bei einem am Kopf der Kolonne gemessenen Druck von 10 bar (absolut) und einem Rücklaufverhältnis von 1,0 betrieben.

In den oberen Kolonnenbereich direkt oberhalb des ersten Reaktionsbodens werden kontinuierlich 30644 kg/h eines Dialkylcarbonat enthaltenden Alkoholgemischs mit 59 Gew.-% MeOH und 41 Gew.-% Dimethylcarbonat zudosiert.

Ein Partialkondensator kondensiert den Dampfstrom am Kopf der Kolonne bei 137°C. Man erhält sowohl 21 kg/h dampfförmiges als auch 21378 kg/h flüssiges Destillat mit einer Zusammensetzung von 84 Gew.-% Methanol und 16 Gew.-% Dimethylcarbonat.

Man erhält 9245 kg/h flüssiges Sumpfprodukt mit einer Zusammensetzung von 99,5 Gew.-% Dimethylcarbonat und 0,5 Gew.-% Methanol. Der Sumpfverdampfer wird mit Heizdampf auf einer Druckstufe von 16 bar bei 183 °C betrieben und hat eine Heizleistung von 10396 kW.

## Patentansprüche

1. Verfahren zur Reinigung von Dialkylcarbonaten in wenigstens einer Destillationskolonne enthaltend wenigstens einen Verstärkungsteil im oberen Teil der Kolonne und wenigstens einen Abtriebsteil im unteren Teil der Kolonne, **dadurch gekennzeichnet, dass**
am Kopf einer Umesterungskolonne ein Dialkylcarbonat-Alkylalkohol-Gemisch entnommen und der Destillationskolonne zugeführt wird,
wobei in der Destillationskolonne zur Aufarbeitung des Dialkylcarbonat-Alkylalkohol-Gemisches eine technische Vorrichtung zur Erhitzung des kolonneninternen Flüssigkeitstromes eingesetzt wird, wobei der kolonneninterne Flüssigkeitsstrom auf ein Temperaturniveau T_{z} erhitzt wird, T_{Z} < T_{SV} ist und T_{SV} das Temperaturniveau ist, welches zum Betrieb eines Sumpfverdampfers benötigt wird
und zur Erhitzung des kolonneninternen Flüssigkeitstromes Energie teilweise oder ganz aus einem anderen chemischen Herstellverfahren gewonnen wird.

2. Verfahren nach Anspruch 1, **dadurch gekennzeichnet, dass** die Energie durch Kondensation in dieser technischen Einrichtung als Kondensationswärme ganz oder teilweise, direkt oder indirekt in das Verfahren zur Erhitzung des kolonneninternen Flüssigkeitstromes eingespeist wird.

3. Verfahren nach Anspruch 1 oder 2, **dadurch gekennzeichnet, dass** diese technische Vorrichtung im Abtriebsteil der Destillationskolonne platziert wird.

4. Verfahren nach Anspruch 1 oder 2, **dadurch gekennzeichnet, dass** diese technische Vorrichtung außerhalb der Destillationskolonne platziert ist.

5. Verfahren nach einem der Ansprüche 1 bis 4, **dadurch gekennzeichnet, dass** die technische Vorrichtung in der oberen Hälfte des Abtriebsteils der Kolonne zum Einsatz kommt.

6. Verfahren nach einem der Ansprüche 1 bis 5, **dadurch gekennzeichnet, dass** die Energie zur Erhitzung des kolonneninternen Flüssigkeitstromes aus einer nachfolgenden Herstellung von Diarylcarbonat gewonnen wird.

7. Verfahren nach Anspruch 6, **dadurch gekennzeichnet, dass** die Energie aus der anfallenden Wärme bei der Kondensation aus wenigstens einem der Schritte in der Diarylcarbonatherstellung ausgewählt aus:
◆ dem oder den in einer ersten Reaktionskolonne(n) der Diarylcarbonatherstellung vorhandenen Zwischenkondensator(en),
◆ dem oder den Kondensator(en) einer zweiten oder weiteren Reaktionskolonne(n) der Diarylcarbonatherstellung,
◆ dem oder den Kondensator(en) zur Kondensation eines Seitenstroms enthaltend das aufgereinigte Diarylcarbonat oder, im Falle, dass eine zweite Diarylcarbonat-Destillationskolonne oder Seitenstromkolonne vorhanden ist, der zweiten Diarylcarbonat-Destillationskolonne oder der Seitenstromkolonne,
◆ dem oder den Kondensator(en) zur Kondensation eines dampfförmigen Seitenstromes der zweiten Zwischensiederkolonne der Diarylcarbonatherstellung
gewonnen wird.

## Claims

1. Process for purifying dialkyl carbonates in at least one distillation column containing at least one enrichment section in the upper part of the column and at least one stripping section in the lower part of the column, **characterized in that**,
at the top of a transesterification column a dialkyl carbonate/alkyl alcohol mixture is taken off and fed to the distillation column,
wherein, in the distillation column for working up the dialkyl carbonate/alkyl alcohol mixture, a technical apparatus for heating the internal liquid stream in the column is used, wherein the internal liquid stream in the column is heated to a temperature level T_{z}, T_{z} < Tₛᵥ, and Tₛᵥ is the temperature level required to operate a bottom vaporizer,
and the energy used to heat the internal liquid stream in the column is partly or entirely recovered from another chemical production process.

2. Process according to Claim 1, **characterized in that** the energy obtained as heat of condensation by condensation in this technical apparatus is fed entirely or partly, directly or indirectly into the process for heating the internal liquid stream in the column.

3. Process according to Claim 1 or 2, **characterized in that** this technical apparatus is positioned in the stripping section of the distillation column.

4. Process according to Claim 1 or 2, **characterized in that** this technical apparatus is positioned outside the distillation column.

5. Process according to any of Claims 1 to 4, **characterized in that** the technical apparatus is used in the upper half of the stripping section of the column.

6. Process according to any of Claims 1 to 5, **characterized in that** the energy for heating the internal liquid stream in the column is obtained from a subsequent preparation of diaryl carbonate.

7. Process according to Claim 6, **characterized in that** the energy from the heat obtained in the condensation is obtained from at least one of the steps in the diaryl carbonate preparation selected from among:
◆ the intermediate condenser(s) present in a first reaction column(s) of the diaryl carbonate preparation,
◆ the condenser(s) of a second or further reaction column(s) of the diaryl carbonate preparation,
◆ the condenser(s) for condensing a side stream containing the purified diaryl carbonate or, in the case of a second diaryl carbonate distillation column or side stream column being present, the second diaryl carbonate distillation column or the side stream column,
◆ the condenser(s) for condensing a gaseous side stream from the second intermediate boiler column of the diaryl carbonate preparation.

## Revendications

1. Procédé pour la purification de carbonates de dialkyle dans au moins une colonne de distillation contenant au moins une partie de concentration dans la partie supérieure de la colonne et au moins une partie d'épuisement dans la partie inférieure de la colonne, **caractérisé en ce qu'**un mélange de carbonate de dialkyle-alcool alkylique est prélevé en tête d'une colonne de transestérification et introduit dans la colonne de distillation,
un dispositif technique pour chauffer le flux de liquide à l'intérieur de la colonne étant utilisé dans la colonne de distillation pour le traitement du mélange de carbonate de dialkyle-alcool alkylique, le flux de liquide à l'intérieur de la colonne étant chauffé à un niveau de température Tz, Tz étant < T_{SV} et Tsv étant le niveau de température qui est nécessaire pour le fonctionnement d'un évaporateur du fond de colonne,
et l'énergie pour le chauffage du flux de liquide à l'intérieur de la colonne étant obtenue partiellement ou totalement à partir d'un autre procédé de préparation chimique.

2. Procédé selon la revendication 1, **caractérisé en ce que** l'énergie obtenue par condensation dans ce dispositif technique en tant que chaleur de condensation est injectée totalement ou partiellement, directement ou indirectement, dans le procédé pour le chauffage du flux de liquide à l'intérieur de la colonne.

3. Procédé selon la revendication 1 ou 2, **caractérisé en ce que** ce dispositif technique est placé dans la partie d'épuisement de la colonne de distillation.

4. Procédé selon la revendication 1 ou 2, **caractérisé en ce que** ce dispositif technique est placé en dehors de la colonne de distillation.

5. Procédé selon l'une quelconque des revendications 1 à 4, **caractérisé en ce que** le dispositif technique est utilisé dans la moitié supérieure de la partie d'épuisement de la colonne.

6. Procédé selon l'une quelconque des revendications 1 à 5, **caractérisé en ce que** l'énergie pour le chauffage du flux de liquide à l'intérieur de la colonne est obtenue à partir d'une préparation consécutive de carbonate de diaryle.

7. Procédé selon la revendication 6, **caractérisé en ce que** l'énergie est obtenue à partir de la chaleur formée lors de la condensation dans au moins une étape de la préparation de carbonate de diaryle choisie parmi :
- le ou les condensateurs intermédiaires présents dans une ou plusieurs colonnes de réaction de la préparation de carbonate de diaryle,
- le ou les condensateurs d'une deuxième ou d'autres colonnes de réaction de la préparation de carbonate de diaryle,
- le ou les condensateurs pour la condensation d'un flux latéral contenant le carbonate de diaryle purifié ou, dans le cas où il existe une deuxième colonne de distillation de carbonate de diaryle ou une deuxième colonne de flux latéral, celui ou ceux de la deuxième colonne de distillation de carbonate de diaryle ou de la deuxième colonne de flux latéral,
- le ou les condensateurs pour la condensation d'un flux latéral sous forme de vapeur de la deuxième colonne d'ébullition intermédiaire de la préparation de carbonate de diaryle.
